# EUROPEAN PATENT APPLICATION

(11) **EP 4 748 860 A1**
(43) Date of publication of application: **27.05.2026**
(21) Application number: 24842444.2
(22) Date of filing: 19.07.2024
(51) Int. Cl.: C07K 16/30, A61K 39/395, A61P 35/00

(54) **ANTI-HER2 NANOBODY, AND PREPARATION METHOD THEREFOR AND USE THEREOF**

(30) Priority: 19.07.2023 CN 202310886901; 18.07.2024 CN 202410968156
(71) Applicant: Kanglitai Biomedical (Qingdao) Co., Ltd., Qingdao, Shandong 266114 (CN)
(72) Inventor: XU, Yinfeng, Qingdao, Shandong 266114 (CN); JIANG, Huichun, Qingdao, Shandong 266114 (CN); ZHENG, Binbin, Qingdao, Shandong 266114 (CN); LI, Tingting, Qingdao, Shandong 266114 (CN); WANG, Lu, Qingdao, Shandong 266114 (CN); LI, Lei, Qingdao, Shandong 266114 (CN); TANG, Tian, Qingdao, Shandong 266114 (CN); JIANG, Lizhong, Qingdao, Shandong 266114 (CN); LI, Zongyan, Qingdao, Shandong 266114 (CN); WANG, Jiangang, Qingdao, Shandong 266114 (CN); WU, Mingyuan, Qingdao, Shandong 266114 (CN)
(74) Representative: Michalski Hüttermann & Partner Patentanwälte mbB
(86) International application number: PCT/CN2024/106555
(87) International publication number: WO 2025/016458

(57) **Abstract**

Provided are an anti-HER2 nanobody, and a preparation method therefor and a use thereof. Also provided are a corresponding anti-HER2 antibody, chimeric antigen receptor, fusion protein, and recombinant protein, an encoding nucleic acid therefor, an expression vector, and a host cell. The anti-HER2 nanobody has high affinity and a simple structure, is easy to prepare, binds to trastuzumab and pertuzumab at different epitopes, and has a wide prospect for application in the fields of tumor treatment, immunodetection and the like.

## Description

### Technical field

The present invention relates to the field of biomedical or biopharmaceutical technology, and more particularly, to an anti-HER2 single-domain antibody, as well as its preparation method and application.

### Background

Human epidermal growth factor receptor 2 (HER2, ERBB2) is a membrane glycoprotein of tyrosine kinase receptor encoded by the *ErbB* gene, and belongs to the member of the epidermal growth factor receptor family. Abnormal expression of HER2 renders normal cells and tissues prone to canceration, leading to tumorigenesis. It is known that amplification of the *HER2* proto-oncogene or overexpression of the HER2 protein have been found in a variety of human tumors, including breast cancer, gastric cancer, colon cancer, ovarian cancer, lung cancer, prostate cancer and so on.

HER2 comprises three structural domains: an extracellular ligand-binding domain (ECD), a short transmembrane region, and an intracellular tyrosine kinase domain. The ECD of HER2 consists of 630 amino acids and contains 4 domains (Domain I-IV). After antibodies bind to different domains of HER2, the mechanisms of action and biological functions they exert are not completely identical, which also provides new insights into the development of the HER2 target. For example, the classic HER2-targeted antibodies Trastuzumab and Pertuzumab bind to different domains: Trastuzumab binds to ECD4, the extracellular domain proximal to the cell membrane of HER2, resulting in inhibition of HER2 signal transduction; Pertuzumab binds to ECD2 of HER2, preventing the homodimerization and heterodimerization of HER2 and HER3. This has become a successful approach for tumor therapy, significantly improving the survival rate and survival time of patients. However, antibody therapy is still faced with problems such as restricted medication and toxic side effects, thus HER2 remains a crucial target for tumor therapy at present.

Compared with traditional monoclonal antibodies, single-domain antibodies have the advantages of low molecular weight, strong penetration, high sensitivity, high specificity and good stability. After entering the body, they can efficiently penetrate cells to rapidly capture antigens for therapeutic purposes. In the field of disease diagnosis, although monoclonal antibodies are also used in tumor imaging, their weak tumor penetration ability and long serum half-life make it difficult to form high-contrast images during tumor imaging, thus limiting their application to a certain extent. Single-domain antibodies have the advantages of easy penetration and rapid renal clearance, and can be labeled with molecules such as fluorescent probes, enzymatic tracers and biotin to serve as tracers. Combined with molecular imaging technology, they can optimize the imaging system, making them ideal candidates for *in vitro* and *in vivo* imaging.

Therefore, there is an urgent need in the art to develop various types of single-domain antibody-based drugs for the specific targeting of HER2.

### Summary of the invention

The objective of the present invention is to provide an anti-HER2 single-domain antibody, as well as its preparation method and application.

Another objective of the present invention is to provide antibodies, chimeric antigen receptors, fusion proteins, recombinant proteins based on the anti-HER2 single-domain antibody, as well as their encoding nucleic acids, expression vectors, host cells and immunoconjugates, etc., and also to provide pharmaceutical compositions comprising the aforesaid active ingredients.

A further objective of the present invention is to provide methods for the prevention and/or treatment of diseases with high HER2 expression, as well as diagnostic methods for diseases with high HER2 expression.

In a first aspect of the present invention, there is provided an anti-HER2 single-domain antibody, wherein the anti-HER2 single-domain antibody has one or more complementarity-determining regions (CDRs) selected from the group consisting of:
(1) CDR1 as set forth in SEQ ID NO: 27, CDR2 as set forth in SEQ ID NO: 28, and CDR3 as set forth in SEQ ID NO: 29;
(2) CDR1 as set forth in SEQ ID NO: 34, CDR2 as set forth in SEQ ID NO: 35, and CDR3 as set forth in SEQ ID NO: 36;
(3) CDR1 as set forth in SEQ ID NO: 40, CDR2 as set forth in SEQ ID NO: 41, and CDR3 as set forth in SEQ ID NO: 42;
(4) CDR1 as set forth in SEQ ID NO: 46, CDR2 as set forth in SEQ ID NO: 47, and CDR3 as set forth in SEQ ID NO: 48;
(5) CDR1 as set forth in SEQ ID NO: 52, CDR2 as set forth in SEQ ID NO: 53, and CDR3 as set forth in SEQ ID NO: 54;
(6) CDR1 as set forth in SEQ ID NO: 57, CDR2 as set forth in SEQ ID NO: 58, and CDR3 as set forth in SEQ ID NO: 59;
(7) CDR1 as set forth in SEQ ID NO: 63, CDR2 as set forth in SEQ ID NO: 64, and CDR3 as set forth in SEQ ID NO: 65;
(8) CDR1 as set forth in SEQ ID NO: 69, CDR2 as set forth in SEQ ID NO: 70, and CDR3 as set forth in SEQ ID NO: 71;
(9) CDR1 as set forth in SEQ ID NO: 74, CDR2 as set forth in SEQ ID NO: 75, and CDR3 as set forth in SEQ ID NO: 76;
(10) CDR1 as set forth in SEQ ID NO: 27, CDR2 as set forth in SEQ ID NO: 28, and CDR3 as set forth in SEQ ID NO: 80;
(11) CDR1 as set forth in SEQ ID NO: 83, CDR2 as set forth in SEQ ID NO: 84, and CDR3 as set forth in SEQ ID NO: 85;
(12) CDR1 as set forth in SEQ ID NO: 57, CDR2 as set forth in SEQ ID NO: 58, and CDR3 as set forth in SEQ ID NO: 89;
(13) CDR1 as set forth in SEQ ID NO: 91, CDR2 as set forth in SEQ ID NO: 92, and CDR3 as set forth in SEQ ID NO: 93;
(14) CDR1 as set forth in SEQ ID NO: 97, CDR2 as set forth in SEQ ID NO: 98, and CDR3 as set forth in SEQ ID NO: 99.

In another preferred embodiment, any one of the aforementioned amino acid sequences further comprises a derivative sequence obtained by optionally adding, deleting, modifying and/or substituting at least one (e.g., 1-3, preferably 1-2, more preferably 1) amino acid and being capable of retaining the ability of specific binding to HER2.

In another preferred embodiment, the derivative sequence obtained by adding, deleting, modifying and/or substituting at least one amino acid and being capable of retaining the ability of specific binding to HER2 is an amino acid sequence having a homology or sequence identity of at least 85%, at least 90%, at least 95%, at least 96%, at least 97%, at least 98% or at least 99%.

In another preferred embodiment, the CDR1, CDR2 and CDR3 are separated in sequence by framework regions FR1, FR2, FR3 and FR4 of a VHH chain, respectively.

In another preferred embodiment, the anti-HER2 single-domain antibody has a structure represented by Formula (I):

FR1-CDR1-FR2-CDR2-FR3-CDR3-FR4 Formula (I)

wherein each "-" independently represents a linker peptide or a peptide bond.

In another preferred embodiment, there is an interloop disulfide bond between the CDR1 region and the CDR3 region of the anti-HER2 single-domain antibody.

In another preferred embodiment, the anti-HER2 single-domain antibody further comprises framework regions (FRs).

In another preferred embodiment, the framework regions (FRs) are one or more FRs selected from the group consisting of:
(1) FR1 as set forth in SEQ ID NO: 30, FR2 as set forth in SEQ ID NO: 31, FR3 as set forth in SEQ ID NO: 32 and FR4 as set forth in SEQ ID NO: 33;
(2) FR1 as set forth in SEQ ID NO: 37, FR2 as set forth in SEQ ID NO: 38, FR3 as set forth in SEQ ID NO: 39 and FR4 as set forth in SEQ ID NO: 33;
(3) FR1 as set forth in SEQ ID NO: 43, FR2 as set forth in SEQ ID NO: 44, FR3 as set forth in SEQ ID NO: 45 and FR4 as set forth in SEQ ID NO: 33;
(4) FR1 as set forth in SEQ ID NO: 37, FR2 as set forth in SEQ ID NO: 49, FR3 as set forth in SEQ ID NO: 50 and FR4 as set forth in SEQ ID NO: 33;
(5) FR1 as set forth in SEQ ID NO: 51, FR2 as set forth in SEQ ID NO: 31, FR3 as set forth in SEQ ID NO: 32 and FR4 as set forth in SEQ ID NO: 33;
(6) FR1 as set forth in SEQ ID NO: 37, FR2 as set forth in SEQ ID NO: 55, FR3 as set forth in SEQ ID NO: 56 and FR4 as set forth in SEQ ID NO: 33;
(7) FR1 as set forth in SEQ ID NO: 60, FR2 as set forth in SEQ ID NO: 61, FR3 as set forth in SEQ ID NO: 62 and FR4 as set forth in SEQ ID NO: 33;
(8) FR1 as set forth in SEQ ID NO: 66, FR2 as set forth in SEQ ID NO: 67, FR3 as set forth in SEQ ID NO: 68 and FR4 as set forth in SEQ ID NO: 33;
(9) FR1 as set forth in SEQ ID NO: 37, FR2 as set forth in SEQ ID NO: 72, FR3 as set forth in SEQ ID NO: 73 and FR4 as set forth in SEQ ID NO: 33;
(10) FR1 as set forth in SEQ ID NO: 77, FR2 as set forth in SEQ ID NO: 78, FR3 as set forth in SEQ ID NO: 79 and FR4 as set forth in SEQ ID NO: 33;
(11) FR1 as set forth in SEQ ID NO: 30, FR2 as set forth in SEQ ID NO: 81, FR3 as set forth in SEQ ID NO: 82 and FR4 as set forth in SEQ ID NO: 33;
(12) FR1 as set forth in SEQ ID NO: 86, FR2 as set forth in SEQ ID NO: 87, FR3 as set forth in SEQ ID NO: 88 and FR4 as set forth in SEQ ID NO: 33;
(13) FR1 as set forth in SEQ ID NO: 30, FR2 as set forth in SEQ ID NO: 61, FR3 as set forth in SEQ ID NO: 90 and FR4 as set forth in SEQ ID NO: 33;
(14) FR1 as set forth in SEQ ID NO: 94, FR2 as set forth in SEQ ID NO: 95, FR3 as set forth in SEQ ID NO: 96 and FR4 as set forth in SEQ ID NO: 33;
(15) FR1 as set forth in SEQ ID NO: 100, FR2 as set forth in SEQ ID NO: 101, FR3 as set forth in SEQ ID NO: 102 and FR4 as set forth in SEQ ID NO: 33.

In another preferred embodiment, the amino acid sequence of the VHH chain of the anti-HER2 single-domain antibody is selected from one or more of SEQ ID NOs: 6-20.

In another preferred embodiment, the anti-HER2 single-domain antibody comprises humanized antibodies, camel-derived antibodies and chimeric antibodies.

In a second aspect of the present invention, there is provided an anti-HER2 antibody, wherein the antibody comprises one or more VHH chains of the anti-HER2 single-domain antibody of the first aspect of the present invention.

In another preferred embodiment, the amino acid sequence of the VHH chain of the anti-HER2 single-domain antibody is selected from one or more of SEQ ID NOs: 6-20.

In another preferred embodiment, the anti-HER2 antibody may be a monomer, a bivalent antibody, and/or a multivalent antibody.

In another preferred embodiment, the anti-HER2 antibody is a bivalent antibody.

In a third aspect of the present invention, there is provided a chimeric antigen receptor (CAR), wherein the CAR comprises an extracellular domain that comprises the anti-HER2 single-domain antibody of the first aspect of the present invention, or the anti-HER2 antibody of the second aspect of the present invention.

In another preferred embodiment, the extracellular domain further comprises a signal peptide.

In another preferred embodiment, the extracellular domain further comprises other heterologous proteins.

In another preferred embodiment, the CAR has a structure represented by Formula Ia:

L-Nb-H-TM-C-CD3ζ (Ia)

wherein,
L is absent or a signal peptide sequence;
Nb is a specific binding domain;
H is absent or a hinge region;
TM is a transmembrane domain;
C is a costimulatory signaling domain;
CD3ζ is a cytoplasmic signaling sequence derived from CD3ζ (including the wild-type form, or mutants/modified forms thereof);
the "-" is a linker peptide or a peptide bond.

In another preferred embodiment, L is a signal peptide of proteins selected from the group consisting of: CD8, GM-CSF, CD4, CD28, CD137, or mutants/modified forms thereof, and combinations thereof.

In another preferred embodiment, Nb targets HER2.

In another preferred embodiment, Nb is an anti-HER2 single-domain antibody.

In another preferred embodiment, H is a hinge region of proteins selected from the group consisting of: CD8, CD28, CD137, IgG, and combinations thereof.

In another preferred embodiment, H is a human IgG1 Fc hinge region.

In another preferred embodiment, TM is a transmembrane domain of proteins selected from the group consisting of: CD28, CD3 epsilon, CD45, CD4, CD5, CD8, CD9, CD16, CD22, CD33, CD37, CD64, CD80, CD86, CD134, CD137, CD154, CD278, CD152, CD279, CD233, or mutants/modified forms thereof, and combinations thereof.

In another preferred embodiment, C is a costimulatory domain of proteins selected from the group consisting of: OX40, CD2, CD7, CD27, CD28, CD30, CD40, CD70, CD134, 4-1BB (CD137), PD-1, Dap10, LIGHT, NKG2C, B7-H3, ICAM-1, LFA-1 (CD11a/CD18), ICOS (CD278), NKG2D, GITR, OX40L, 2B4, TLR, or mutants/modified forms thereof, and combinations thereof.

In another preferred embodiment, the CAR comprises an intracellular domain of a cytokine.

In another preferred embodiment, the cytokine comprises: interleukins (ILs), interferons (IFNs), tumor necrosis factors (TNFs), colony-stimulating factors (CSFs), growth factors, chemokines, or combinations thereof; preferably, the cytokine is an interleukin.

In another preferred embodiment, the CAR comprises an intracellular domain of an interleukin.

In another preferred embodiment, the interleukin is selected from the group consisting of: IL-12, IL-2, IL-15, IL-21, and combinations thereof; preferably IL-12.

In a fourth aspect of the present invention, there is provided a fusion protein, wherein the fusion protein comprises:
(Z1) a first protein, wherein the first protein comprises: the anti-HER2 single-domain antibody of the first aspect of the present invention, the anti-HER2 antibody of the second aspect of the present invention, or an active fragment thereof;
(Z2) a second protein, wherein the second protein comprises a cytokine; and
(Z3) an optional linker located between the first protein and the second protein.

In another preferred embodiment, the cytokine comprises: interleukins (ILs), interferons (IFNs), tumor necrosis factors (TNFs), colony-stimulating factors (CSFs), growth factors, chemokines, or combinations thereof.

In another preferred embodiment, the second protein is an interleukin.

In another preferred embodiment, the first protein is the anti-HER2 single-domain antibody of the first aspect of the present invention, and the second protein is an interleukin.

In another preferred embodiment, the interleukin is selected from the group consisting of: IL-12, IL-2, IL-15, IL-21, and combinations thereof.

In another preferred embodiment, the interleukin is IL-12.

In a fifth aspect of the present invention, there is provided a recombinant protein, wherein the recombinant protein comprises:
(i) the anti-HER2 single-domain antibody of the first aspect of the present invention, the anti-HER2 antibody of the second aspect of the present invention, the fusion protein of the fourth aspect of the present invention, or an active fragment thereof; and
(ii) an optional tag sequence that facilitates expression and/or purification.

In another preferred embodiment, the tag comprises an Fc tag, an HA tag, a GGGS sequence, a FLAG tag, a Myc tag, a 6His tag, or combinations thereof.

In another preferred embodiment, the recombinant protein specifically binds to HER2.

In another preferred embodiment, the recombinant protein is a monomer, a dimer, or a multimer.

In another preferred embodiment, the tag is an Fc tag.

In a sixth aspect of the present invention, there is provided a polynucleotide, wherein the polynucleotide encodes a protein selected from the group consisting of: the anti-HER2 single-domain antibody of the first aspect of the present invention, the anti-HER2 antibody of the second aspect of the present invention, the chimeric antigen receptor of the third aspect of the present invention, the fusion protein of the fourth aspect of the present invention, the recombinant protein of the fifth aspect of the present invention, and combinations thereof.

In another preferred embodiment, the polynucleotide is RNA, DNA or cDNA.

In a seventh aspect of the present invention, there is provided an expression vector, wherein the expression vector comprises the polynucleotide of the sixth aspect of the present invention.

In another preferred embodiment, the expression vector is selected from the group consisting of: DNA, RNA, viral vectors, plasmids, transposons, other gene transfer systems, and combinations thereof. Preferably, the expression vector comprises viral vectors, such as lentiviruses, adenoviruses, AAV viruses, retroviruses, or combinations thereof.

In another preferred embodiment, the expression vector is selected from the group consisting of: pTomo lentiviral vector, plenti, pLVTH, pLJM1, pHCMV, pLBS.CAG, pHR, and pLV, etc.

In another preferred embodiment, the expression vector is pcDNA3.1 vector, pMES4 vector, or pABG1 vector (including pABG1-Fc vector).

In another preferred embodiment, the expression vector further comprises elements selected from the group consisting of: promoters, transcriptional enhancement element WPRE, and long terminal repeats (LTRs), etc.

In an eighth aspect of the present invention, there is provided a host cell, wherein the host cell comprises the expression vector of the seventh aspect of the present invention, or has the polynucleotide of the sixth aspect of the present invention integrated into its genome.

In another preferred embodiment, the host cell comprises prokaryotic cells or eukaryotic cells.

In another preferred embodiment, the host cell is selected from the group consisting of: *Escherichia coli,* yeast cells, and mammalian cells.

In a ninth aspect of the present invention, there is provided an engineered immune cell, wherein the engineered immune cell comprises the expression vector of the seventh aspect of the present invention, or has the exogenous polynucleotide of the sixth aspect of the present invention integrated into its genome, or expresses the chimeric antigen receptor of the third aspect of the present invention.

In another preferred embodiment, the engineered immune cell is selected from the group consisting of:
(i) chimeric antigen receptor αβT cells (CAR-T cells);
(ii) chimeric antigen receptor γδT cells (CAR-T cells);
(iii) chimeric antigen receptor NKT cells (CAR-NKT cells);
(iv) chimeric antigen receptor NK cells (CAR-NK cells).

In another preferred embodiment, the engineered immune cell comprises autologous or allogeneic αβT cells, γδT cells, NKT cells, NK cells, or combinations thereof.

In another preferred embodiment, the engineered immune cell is a CAR-T cell.

In a tenth aspect of the present invention, there is provided a method for producing an anti-HER2 single-domain antibody, wherein the method comprises the steps of:
(a) culturing the host cell of the eighth aspect of the present invention under conditions suitable for producing a single-domain antibody, thereby obtaining a culture containing the anti-HER2 single-domain antibody;
(b) isolating and/or recovering the anti-HER2 single-domain antibody from the culture; and
(c) optionally, purifying and/or modifying the anti-HER2 single-domain antibody obtained in step (b).

In an eleventh aspect of the present invention, there is provided an immunoconjugate, wherein the immunoconjugate comprises:
(a) the anti-HER2 single-domain antibody of the first aspect of the present invention, the anti-HER2 antibody of the second aspect of the present invention, the fusion protein of the fourth aspect of the present invention, or the recombinant protein of the fifth aspect of the present invention; and
(b) a coupling moiety selected from the group consisting of: detectable labels, drugs, cytokines, radionuclides, enzymes, gold nanoparticles/nanorods, magnetic nanoparticles, viral capsid proteins or VLPs, and combinations thereof.

In another preferred embodiment, the moiety (a) is the anti-HER2 single-domain antibody of the first aspect of the present invention, or the anti-HER2 antibody of the second aspect of the present invention.

In another preferred embodiment, the moiety (a) is conjugated to the coupling moiety via chemical bonds or linkers.

In another preferred embodiment, the radionuclide comprises:
(i) diagnostic isotopes selected from the group consisting of: Tc-99m, Ga-68, F-18, I-123, I-125, I-131, In-111, Ga-67, Cu-64, Zr-89, C-11, Lu-177, Re-188, and combinations thereof; and/or
(ii) therapeutic isotopes selected from the group consisting of: Lu-177, Y-90, Ac-225, As-211, Bi-212, Bi-213, Cs-137, Cr-51, Co-60, Dy-165, Er-169, Fm-255, Au-198, Ho-166, I-125, 1-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133, Yb-169, Yb-177, and combinations thereof.

In another preferred embodiment, the coupling moiety is a drug or a toxin.

In another preferred embodiment, the drug is a therapeutic agent targeting HER2-overexpressing diseases.

In another preferred embodiment, the HER2-overexpressing disease is selected from the group consisting of: breast cancer, gastric cancer, colorectal cancer, ovarian cancer, lung cancer, prostate cancer, liver cancer, renal tumor, small intestinal cancer, large intestinal cancer, cholangiocarcinoma, cervical cancer, lymphoma, esophageal cancer, and combinations thereof.

In another preferred embodiment, the drug is a cytotoxic agent.

In another preferred embodiment, the cytotoxic agent is selected from the group consisting of: anti-tubulin agents, DNA minor groove binders, DNA replication inhibitors, alkylating agents, antibiotics, folate antagonists, antimetabolites, chemosensitizers, topoisomerase inhibitors, vinca alkaloids, and combinations thereof.

Particularly useful examples of cytotoxic agent classes include, for example, DNA minor groove binders, DNA alkylating agents, and tubulin inhibitors. Typical cytotoxic agents include, for example, Auristatins, Camptothecins, Duocarmycins, Etoposides, Maytansines and Maytansinoids (e.g., DM1 and DM4), Taxanes, Benzodiazepines or Benzodiazepine-containing drugs (e.g., pyrrolo[1,4]benzodiazepines (PBDs), Indolinobenzodiazepines and Oxazolidinobenzodiazepines), Vinca alkaloids, or combinations thereof.

In another preferred embodiment, the toxin is selected from the group consisting of: Auristatins (e.g., Auristatin E, Auristatin F, MMAE and MMAF), chlortetracycline, maytansinoids, ricin, ricin A-chain, combretastatins, duocarmycins, dolastatins, doxorubicin, daunorubicin, paclitaxel, cisplatin, cc1065, ethidium bromide, mitomycin, etoposide, tenoposide, vincristine, vinblastine, colchicine, dioxanthracenedione, actinomycin, diphtheria toxin, Pseudomonas exotoxin (PE) A, PE40, abrin, abrin A-chain, modeccin A-chain, α-sarcin, gelonin, mitogellin, restrictocin, phenomycin, enomycin, curicin, croton toxin, calicheamicin, Sapaonaria officinalis inhibitors, glucocorticoids, and combinations thereof.

In another preferred embodiment, the coupling moiety is a detectable label.

In another preferred embodiment, the coupling moiety is selected from the group consisting of: fluorescent or luminescent labels, radioactive labels, MRI (magnetic resonance imaging) or CT (computed tomography) contrast agents, or enzymes capable of generating detectable products, radionuclides, biotoxins, cytokines (e.g., IL-2, etc.), antibodies, antibody Fc fragments, antibody scFv fragments, gold nanoparticles/nanorods, viral particles, liposomes, magnetic nanoparticles, prodrug-activating enzymes (e.g., DT-diaphorase (DTD) or biphenyl hydrolase-like protein (BPHL)), and nanoparticles of any form.

In another preferred embodiment, the immunoconjugate comprises: multivalent (e.g., bivalent) VHH chains of the anti-HER2 single-domain antibody of the first aspect of the present invention.

In another preferred embodiment, the term "multivalent" means that the amino acid sequence of the immunoconjugate comprises multiple repeated identical or different VHH chains of the anti-HER2 single-domain antibody of the first aspect of the present invention.

In a twelfth aspect of the present invention, there is provided a use of an active ingredient, wherein the active ingredient is selected from the group consisting of: the anti-HER2 single-domain antibody of the first aspect of the present invention, the anti-HER2 antibody of the second aspect of the present invention, the chimeric antigen receptor of the third aspect of the present invention, the fusion protein of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, and combinations thereof, and the active ingredient is used for preparing:
(a) a medicament for preventing and/or treating HER2-overexpressing diseases;
(b) a reagent for detecting HER2-overexpressing diseases.

In another preferred embodiment, the reagent is a diagnostic reagent, preferably, the diagnostic reagent is a detection strip or a detection plate.

In another preferred embodiment, the diagnostic reagent is used for: detecting the HER2 protein or fragments thereof in a sample.

In another preferred embodiment, the HER2-overexpressing disease is selected from the group consisting of: breast cancer, gastric cancer, colorectal cancer, ovarian cancer, lung cancer, prostate cancer, liver cancer, renal tumor, small intestinal cancer, large intestinal cancer, cholangiocarcinoma, cervical cancer, lymphoma, esophageal cancer, and combinations thereof.

In a thirteenth aspect of the present invention, there is provided a method for *in vitro* detection of HER2 protein or fragments thereof in a sample, wherein the method comprises the steps of:
(1) *in vitro,* contacting the sample with the anti-HER2 single-domain antibody of the first aspect of the present invention, the anti-HER2 antibody of the second aspect of the present invention, the chimeric antigen receptor of the third aspect of the present invention, the fusion protein of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof;
(2) detecting whether an antigen-antibody complex is formed, wherein the formation of the complex indicates the presence of HER2 protein or fragments thereof in the sample.

In another preferred embodiment, the detection includes diagnostic or nondiagnostic detection.

In a fourteenth aspect of the present invention, there is provided a pharmaceutical composition comprising:
(i) the anti-HER2 single-domain antibody of the first aspect of the present invention, the anti-HER2 antibody of the second aspect of the present invention, the chimeric antigen receptor of the third aspect of the present invention, the fusion protein of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof, as an active ingredient; and
(ii) a pharmaceutically acceptable carrier, diluent or excipient.

In another preferred embodiment, the dosage form of the pharmaceutical composition is selected from the group consisting of: injections and lyophilized preparations.

In another preferred embodiment, the pharmaceutical composition comprises 0.01-99.99% of the anti-HER2 single-domain antibody of the first aspect of the present invention, the anti-HER2 antibody of the second aspect of the present invention, the chimeric antigen receptor of the third aspect of the present invention, the fusion protein of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof, and 0.01-99.99% of a pharmaceutically acceptable carrier, wherein the percentages are calculated based on the total mass of the pharmaceutical composition.

In another preferred embodiment, the concentration of the engineered immune cells in the active ingredient is 1×10³-1×10⁸ cells/mL, preferably 1×10⁴-1×10⁷ cells/mL.

In a fifteenth aspect of the present invention, there is provided a kit comprising:
(1) a first container containing the anti-HER2 single-domain antibody of the first aspect of the present invention, the anti-HER2 antibody of the second aspect of the present invention, the chimeric antigen receptor of the third aspect of the present invention, the fusion protein of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof; and/or
(2) a second container containing a secondary antibody against the contents of the first container;
or,
the kit comprises a detection plate, the detection plate comprising: a substrate (support plate) and a test strip, wherein the test strip contains the anti-HER2 single-domain antibody of the first aspect of the present invention, the anti-HER2 antibody of the second aspect of the present invention, the fusion protein of the fourth aspect of the present invention, the recombinant protein of the fifth aspect of the present invention, the immunoconjugate of the eleventh aspect of the present invention, or combinations thereof.

In another preferred embodiment, the kit further comprises an instruction manual, according to which the kit is used for non-invasive detection of HER2 expression in a subject to be tested.

In another preferred embodiment, the kit is used for detection of HER2-overexpressing diseases.

In another preferred embodiment, the HER2-overexpressing disease is selected from the group consisting of: breast cancer, gastric cancer, colorectal cancer, ovarian cancer, lung cancer, prostate cancer, liver cancer, renal tumor, small intestinal cancer, large intestinal cancer, cholangiocarcinoma, cervical cancer, lymphoma, esophageal cancer, and combinations thereof.

In a sixteenth aspect of the present invention, there is provided a method for preventing and/or treating HER2-overexpressing diseases, wherein the method comprises: administering the anti-HER2 single-domain antibody of the first aspect of the present invention, the anti-HER2 antibody of the second aspect of the present invention, the chimeric antigen receptor of the third aspect of the present invention, the fusion protein of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, the pharmaceutical composition of the fourteenth aspect of the present invention, or combinations thereof, to a subject in need thereof.

In another preferred embodiment, the subject comprises a mammal, such as a human.

In another preferred embodiment, the HER2-overexpressing disease is selected from the group consisting of: breast cancer, gastric cancer, colorectal cancer, ovarian cancer, lung cancer, prostate cancer, liver cancer, renal tumor, small intestinal cancer, large intestinal cancer, cholangiocarcinoma, cervical cancer, lymphoma, esophageal cancer, and combinations thereof.

In another preferred embodiment, the engineered immune cells or the CAR immune cells contained in the pharmaceutical composition are cells derived from the subject (autologous cells).

In another preferred embodiment, the engineered immune cells or the CAR immune cells contained in the pharmaceutical composition are cells derived from a healthy individual (allogeneic cells).

In another preferred embodiment, the method may be used in combination with other therapeutic methods.

In another preferred embodiment, the other therapeutic methods include chemotherapy, radiotherapy, targeted therapy, and the like.

In a seventeenth aspect of the present invention, there is provided a diagnostic method for HER2-overexpressing diseases, comprising the steps of:
(i) obtaining a sample from a subject to be diagnosed, and contacting the sample with the anti-HER2 single-domain antibody of the first aspect of the present invention, the anti-HER2 antibody of the second aspect of the present invention, the chimeric antigen receptor of the third aspect of the present invention, the fusion protein of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof; and
(ii) detecting whether an antigen-antibody complex is formed, wherein the formation of the complex indicates that the subject is a confirmed patient with a HER2-overexpressing disease.

In another preferred embodiment, the sample is a blood sample, a throat swab sample, or a sample from other tissues and organs.

In another preferred embodiment, the HER2-overexpressing disease is selected from the group consisting of: breast cancer, gastric cancer, colorectal cancer, ovarian cancer, lung cancer, prostate cancer, liver cancer, renal tumor, small intestinal cancer, large intestinal cancer, cholangiocarcinoma, cervical cancer, lymphoma, esophageal cancer, and combinations thereof.

In an eighteenth aspect of the present invention, there is provided a method for preparing a recombinant polypeptide, wherein the recombinant polypeptide is the anti-HER2 single-domain antibody of the first aspect of the present invention, the anti-HER2 antibody of the second aspect of the present invention, the chimeric antigen receptor of the third aspect of the present invention, the fusion protein of the fourth aspect of the present invention, the recombinant protein of the fifth aspect of the present invention, or combinations thereof, and the method comprises:
(a) culturing the host cell of the eighth aspect of the present invention under conditions suitable for expression; and
(b) isolating the recombinant polypeptide from the culture.

It should be understood that within the scope of the present invention, the above-mentioned technical features of the present invention and the technical features specifically described in the following (such as the examples) can be combined with each other to form a new or preferred technical solution, which is not redundantly repeated one by one herein due to space limitation.

### Description of the drawings

Figure 1 shows the identification of purified antigen protein by SDS-PAGE gel electrophoresis. Lane 1: non-reduced SDS-PAGE of HER2 protein; Lane 2: reduced SDS-PAGE of HER2 protein; M: protein molecular weight marker.
Figure 2 shows the identification of purified single-domain antibodies by non-reduced SDS-PAGE gel electrophoresis. Lanes 1-11 (left): corresponding to 11 single-domain antibodies respectively (HER2-1, HER2-2, HER2-3, HER2-4, HER2-5, HER2-8, HER2-10, HER2-11, HER2-16, HER2-32 and HER2-34); Lane 12 (left): protein molecular weight marker; Lanes 1-4 (right): corresponding to 4 single-domain antibodies respectively (HER2-37, HER2-38, HER2-40 and HER2-41); Lane 5 (right): protein molecular weight marker.
Figure 3 shows the identification of purified HER2-Fc recombinant antigen protein by SDS-PAGE gel electrophoresis. Lane 7: non-reduced SDS-PAGE of HER2-Fc protein; Lane 6: protein molecular weight marker.
Figures 4-5 show the identification of single-domain antibody affinity, wherein Figure 4 involves HER2-1, HER2-2, HER2-3, HER2-4, HER2-5, HER2-8, HER2-10 and HER2-11; Figure 5 involves HER2-16, HER2-32, HER2-34, HER2-37, HER2-38, HER2-40 and HER2-41.
Figure 6 shows the fitting curves corresponding to the single-concentration affinity screening test of single-domain antibodies.
Figure 7 shows the comparison results of the single-concentration affinity screening test of single-domain antibodies using surface plasmon resonance (SPR) technology.
Figures 8-9 show the binding activity of 15 anti-HER2 single-domain antibodies to HER2 expressed on SKBR3 cells detected by FACS (identification of anti-HER2 single-domain antibody affinity at the cellular level). Among them, Figure 8 involves HER2-2, HER2-3, HER2-8, HER2-11, HER2-38, HER2-5, HER2-10 and HER2-41; Figure 9 involves HER2-1, HER2-4, HER2-16, HER2-32, HER2-34, HER2-37 and HER2-40.
Figure 10 shows the identification of purified humanized single-domain antibodies by non-reduced SDS-PAGE gel electrophoresis. Lane 1: HER2-3-hFc; Lane 2: HER2-8-hFc; Lane 3: HER2-10-hFc; Lane 4: HER2-11-hFc; Lane 5: HER2-38-hFc; Lane 6: protein molecular weight marker.
Figure 11 shows the identification of purified antibody protein by SDS-PAGE gel electrophoresis. Lane 1: non-reduced SDS-PAGE of Trastuzumab; Lane 2: protein molecular weight marker; Lane 3: reduced SDS-PAGE of Trastuzumab.
Figure 12 shows the identification of purified antibody protein by SDS-PAGE gel electrophoresis. Lane 1: non-reduced SDS-PAGE of Pertuzumab; Lane 2: protein molecular weight marker; Lane 3: reduced SDS-PAGE of Pertuzumab.
Figure 13 shows the binding activity analysis of 5 purified single-domain antibody Fc fusion proteins and the positive control Trastuzumab to HER2 protein by ELISA (affinity identification of single-domain antibody Fc fusion proteins).
Figure 14 shows the results of affinity determination of single-domain antibody Fc fusion proteins at the cellular level.
Figure 15 shows the results of specificity detection of anti-HER2 single-domain antibody Fc fusion proteins.
Figure 16 shows the epitope verification of single-domain antibodies and Trastuzumab.
Figure 17 shows the epitope verification of single-domain antibodies and Pertuzumab.
Figure 18 shows the endocytosis rate of single-domain antibodies in SKBR3 cells.

### Detailed description

After extensive and in-depth research and numerous screenings, the inventors of the present invention have unexpectedly obtained anti-HER2 single-domain antibodies with high affinity and high specificity for the first time. Specifically, the present invention designed and constructed a corresponding vector carrying the gene encoding the extracellular domain of HER2 to obtain the extracellular domain protein of HER2. This protein was then used to immunize healthy adult alpacas, thereby generating a high-quality immune library with a capacity of 10¹¹. Phage display technology was employed to screen the immune library, resulting in the acquisition of a class of HER2-specific single-domain antibodies. The anti-HER2 single-domain antibodies of the present invention are characterized by high affinity, simple structure, and ease of preparation, and they bind to different antigenic epitopes from those targeted by Trastuzumab and Pertuzumab, thus holding broad application prospects in fields such as tumor therapy and immunoassay. For instance, the HER2-targeted single-domain antibodies developed by the present invention can serve as a novel therapeutic modality for the targeted treatment of diseases such as breast cancer, gastric cancer, colorectal cancer, ovarian cancer, lung cancer, prostate cancer, liver cancer, renal tumor, small intestine cancer, large intestine cancer, cholangiocarcinoma, cervical cancer, lymphoma and esophageal cancer. On this basis, the present invention has been completed.

### Terms

To facilitate understanding of the present disclosure, certain terms are first defined. As used herein, unless otherwise explicitly specified in the text, each of the following terms shall have the meaning given below.

**Definitions of abbreviations and key terms**

| **Abbreviation** | **English Full Name** |
|---|---|
| AHC | anti-human IgG (Fc) capture antibody |
| CDR | complementary determining region |
| EC₅₀ | concentration for 50% of maximal effect |
| FBS | fetal bovine serum |
| Fc | fragment of crystalization |
| FITC | fluorescein isothiocyanate |
| HRP | horseradish peroxidase |
| IC₅₀ | half maximal inhibitory concentration |
| IFN | interferon |
| IgG | immunoglobulin G |
| kD | kilodalton |
| mAb | monoclonal antibody |
| MFI | median fluorescent intensity |
| NK | nature killer |
| nM | nanomole |
| OD | optical density |
| PBS | phosphate-buffered saline |
| PCR | polymerase chain reaction |
| PEG | polyethylene glycol |
| SDS | sodium dodecyl sulface |
| SPR | surface plasmon resonance |
| TMB | 3,3',5,5'-tetramethylbenzidine |

The term "about" may refer to a value or composition within an acceptable error range of a specific value or composition determined by those of ordinary skill in the art, which will depend in part on how the value or composition is measured or determined.

The term "administration" refers to the physical introduction of the product of the present invention into a subject using any one of various methods and delivery systems known to those of ordinary skill in the art, including intravenous, intratumoral, intramuscular, subcutaneous, intraperitoneal, intraspinal, or other parenteral routes of administration, such as by injection or infusion.

The terms "nanobody", "single-domain antibody", "antibody of the present invention", "single-domain antibody of the present invention" and the like can be used interchangeably, all referring to the antibody of the first aspect of the present invention that specifically recognizes and binds to HER2 protein (including human HER2 protein).

The antibody numbers of the single-domain antibodies of the present invention and their corresponding sequence numbers are shown in Table A below.

**Table A**

| Antibody Number | CDR1 | CDR2 | CDR3 | FR1 | FR2 | FR3 | FR4 | VHH Chain Amino Acid Sequence Number |
|---|---|---|---|---|---|---|---|---|
| HER2-2 | 27 | 28 | 29 | 30 | 31 | 32 | 33 | 7 |
| HER2-8 | 34 | 35 | 36 | 37 | 38 | 39 | 33 | 11 |
| HER2-10 | 40 | 41 | 42 | 43 | 44 | 45 | 33 | 12 |
| HER2-11 | 46 | 47 | 48 | 37 | 49 | 50 | 33 | 13 |
| HER2-40 | 27 | 28 | 29 | 51 | 31 | 32 | 33 | 19 |
| HER2-37 | 52 | 53 | 54 | 37 | 55 | 56 | 33 | 17 |
| HER2-38 | 57 | 58 | 59 | 60 | 61 | 62 | 33 | 18 |
| HER2-16 | 63 | 64 | 65 | 66 | 67 | 68 | 33 | 14 |
| HER2-41 | 69 | 70 | 71 | 37 | 72 | 73 | 33 | 20 |
| HER2-1 | 74 | 75 | 76 | 77 | 78 | 79 | 33 | 6 |
| HER2-3 | 27 | 28 | 80 | 30 | 81 | 82 | 33 | 8 |
| HER2-4 | 83 | 84 | 85 | 86 | 87 | 88 | 33 | 9 |
| HER2-5 | 57 | 58 | 89 | 30 | 61 | 90 | 33 | 10 |
| HER2-32 | 91 | 92 | 93 | 94 | 95 | 96 | 33 | 15 |
| HER2-34 | 97 | 98 | 99 | 100 | 101 | 102 | 33 | 16 |

| | | | | | | | | |
|---|---|---|---|---|---|---|---|---|
| Note: each value in the table represents a sequence number; i.e., "1" denotes "SEQ ID NO: 1". The sequence numbers of CDR1, CDR2, CDR3, FR1, FR2, FR3 and FR4 shown in the table refer to the numbers of their respective amino acid sequences. | | | | | | | | |

As used herein, the term "antibody" or "immunoglobulin" refers to a heterotetrameric glycoprotein of approximately 150,000 daltons with the same structural characteristics, consisting of two identical light chains (L) and two identical heavy chains (H). Each light chain is linked to a heavy chain by a covalent disulfide bond, and the number of inter-heavy chain disulfide bonds varies among different immunoglobulin isotypes. Each heavy chain and light chain also contains regularly spaced intrachain disulfide bonds. One end of each heavy chain has a variable region (VH), followed by multiple constant regions. One end of each light chain has a variable region (VL), and the other end has a constant region; the constant region of the light chain is opposite to the first constant region of the heavy chain, and the variable region of the light chain is opposite to the variable region of the heavy chain. Specific amino acid residues form an interface between the variable regions of the light and heavy chains.

As used herein, the terms "single-domain antibody", "VHH", "nanobody", "single domain antibody (sdAb) or nanobody" have the same meaning and can be used interchangeably, referring to a single-domain antibody (VHH) composed of only one heavy chain variable region cloned from the variable region of an antibody heavy chain, which is the smallest antigen-binding fragment with complete function. Usually, after obtaining an antibody that naturally lacks the light chain and heavy chain constant region 1 (CH1), the variable region of the antibody heavy chain is cloned to construct a single-domain antibody (VHH) composed of only one heavy chain variable region.

As used herein, the term "variable" means that certain parts of the variable regions in antibodies differ in sequence, which confers the binding capacity and specificity of various specific antibodies to their specific antigens. However, variability is not uniformly distributed throughout the antibody variable regions. It is concentrated in three segments in the variable regions of light and heavy chains called complementarity-determining regions (CDRs) or hypervariable regions. The more conserved parts in the variable regions are called framework regions (FRs). The variable regions of natural heavy and light chains each contain four FR regions, which are roughly in a β-sheet conformation, connected by three CDRs forming connecting loops, and may form part of a β-sheet structure in some cases. The CDRs in each chain are closely adjacent through the FR regions and together with the CDRs of the other chain form the antigen-binding site of the antibody (see Kabat et al., NIH Publ. No. 91-3242, Volume I, pp. 647-669 (1991)). The constant regions are not directly involved in the binding of the antibody to the antigen, but they exhibit different effector functions, such as participating in antibody-dependent cellular cytotoxicity.

As known to those skilled in the art, immunoconjugates and fusion expression products include conjugates formed by combining drugs, toxins, cytokines, radionuclides, enzymes, and other diagnostic or therapeutic molecules with the antibodies or fragments thereof of the present invention. The present invention also encompasses cell surface markers or antigens that bind to the HER2-targeted single-domain antibodies or fragments thereof.

As used herein, the terms "hypervariable region", "highly variable region", "complementary determining region" and "complementarity-determining region (CDR)" can be used interchangeably.

In one preferred embodiment of the present invention, the heavy chain variable region of the single-domain antibody or antibody comprises three complementarity-determining regions, namely CDR1, CDR2, and CDR3.

In one preferred embodiment of the present invention, the heavy chain of the single-domain antibody or antibody comprises the aforementioned heavy chain variable region and a heavy chain constant region.

In the present invention, the terms "single-domain antibody of the present invention", "antibody of the present invention", "protein of the present invention", or "polypeptide of the present invention" can be used interchangeably, all referring to polypeptides that specifically bind to the HER2 protein, such as proteins or polypeptides having a heavy chain variable region. They may or may not contain an initiator methionine.

The present invention also provides other proteins or fusion expression products having the antibody of the present invention. Specifically, the present invention includes any protein or protein conjugate and fusion expression product (i.e., immunoconjugate and fusion expression product) having a heavy chain containing a variable region, as long as the variable region has the same or at least 90% homology, preferably at least 95% homology, with the heavy chain variable region of the antibody of the present invention.

In general, the antigen-binding properties of an antibody can be defined by three specific regions located within the heavy chain variable region, known as complementarity-determining regions (CDRs), which divide the variable region into four framework regions (FRs). The amino acid sequences of the four FRs are relatively conserved and do not directly participate in antigen-binding reactions. These CDRs form loop structures that are spatially brought together by the β-sheets formed by the intervening FRs; the CDRs on the heavy chain and the CDRs on the corresponding light chain constitute the antigen-binding site of the antibody. The amino acid sequences of antibodies of the same class can be compared to identify the amino acid residues that make up the FR or CDR regions.

The heavy chain variable regions of the single-domain antibodies or antibodies of the present invention are of particular interest, as they are at least partially responsible for antigen binding. Therefore, the present invention encompasses molecules comprising antibody heavy chain variable regions with CDRs, provided that the CDRs of said molecules share at least 90% homology (preferably at least 95% homology, most preferably at least 98% homology) with the CDRs identified herein.

The present invention includes not only intact antibodies, but also immunologically active antibody fragments as well as fusion proteins formed by linking the antibodies to other sequences. Accordingly, the present invention also encompasses fragments, derivatives and analogs of the said antibodies.

As used herein, the terms "fragment", "derivative", and "analog" refer to polypeptides that substantially retain the same biological function or activity as the antibody of the present invention. The polypeptide fragments, derivatives, or analogs of the present invention may be (i) polypeptides with one or more conservative or non-conservative amino acid residues (preferably conservative amino acid residues) substituted, where the substituted amino acid residues may or may not be encoded by the genetic code; (ii) polypeptides with substitution groups in one or more amino acid residues; (iii) polypeptides formed by fusing a mature polypeptide with another compound (such as a compound that extends the half-life of the polypeptide, e.g., polyethylene glycol); or (iv) polypeptides formed by fusing additional amino acid sequences to this polypeptide sequence (e.g., a leader sequence, a secretion sequence, a sequence used for purifying the polypeptide, a proprotein sequence, or a fusion protein formed with a 6His tag). According to the teachings herein, these fragments, derivatives, and analogs are within the scope well known to those skilled in the art.

The antibody of the present invention refers to a polypeptide that has HER2 protein-binding activity and includes the above-mentioned CDR regions. The term also includes variant forms of polypeptides containing the above-mentioned CDR regions that have the same function as the antibody of the present invention. These variant forms include (but are not limited to): deletion, insertion, and/or substitution of one or more (usually 1-50, preferably 1-30, more preferably 1-20, most preferably 1-10) amino acids, and addition of one or several (usually within 20, preferably within 10, more preferably within 5) amino acids at the C-terminus and/or N-terminus. For example, in the art, substitution with amino acids of similar or analogous properties usually does not change the function of the protein. Similarly, adding one or several amino acids at the C-terminus and/or N-terminus usually does not change the function of the protein. The term also includes active fragments and active derivatives of the antibody of the present invention.

Variant forms of the polypeptide include: homologous sequences, conservative variants, allelic variants, natural mutants, induced mutants, proteins encoded by DNA that can hybridize with the DNA encoding the antibody of the present invention under high or low stringency conditions, and polypeptides or proteins obtained using antiserum against the antibody of the present invention.

The present invention also provides other polypeptides, such as fusion proteins comprising the antibody or fragments thereof. In addition to almost full-length polypeptides, the present invention also includes fragments of the antibody of the present invention. Usually, the fragment has at least about 50 consecutive amino acids, preferably at least about 50 consecutive amino acids, more preferably at least about 80 consecutive amino acids, most preferably at least about 100 consecutive amino acids of the antibody of the present invention.

In the present invention, a "conservative variant of the antibody of the present invention" refers to a polypeptide formed by substituting at most 10, preferably at most 8, more preferably at most 5, most preferably at most 3 amino acids with amino acids of similar or analogous properties compared to the amino acid sequence of the antibody of the present invention. These conservative variant polypeptides are preferably produced by amino acid substitution according to Table B.

**Table B**

| Initial Residue | Representative Substitution | Preferred Substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention further provides polynucleotide molecules encoding the aforementioned antibodies, fragments thereof, or fusion proteins thereof. The polynucleotides of the present invention may be in the form of DNA or RNA. DNA forms include cDNA, genomic DNA, or synthetic DNA. DNA may be singlestranded or double-stranded. DNA may be either the coding strand or the non-coding strand.

Polynucleotides encoding the mature polypeptides of the present invention include: coding sequences that encode only the mature polypeptides; coding sequences of the mature polypeptides combined with various additional coding sequences; coding sequences of the mature polypeptides (optionally with additional coding sequences) together with non-coding sequences.

The term "polynucleotide encoding a polypeptide" may refer to a polynucleotide that contains the coding sequence for the polypeptide, or refer to a polynucleotide that further contains additional coding and/or non-coding sequences.

The present invention also relates to polynucleotides that hybridize to the aforementioned sequences and share at least 50% identity, preferably at least 70% identity, and more preferably at least 80% identity with said sequences. In particular, the present invention relates to polynucleotides capable of hybridizing to the polynucleotides of the present invention under stringent conditions. In the present invention, "stringent conditions" refer to: (1) hybridization and elution performed at low ionic strength and high temperature, e.g., 0.2×SSC, 0.1% SDS, 60°C; (2) hybridization conducted in the presence of denaturants, e.g., 50% (v/v) formamide, 0.1% calf serum/0.1% Ficoll, 42°C, etc.; or (3) hybridization occurring only when the identity between the two sequences is at least 90%, more preferably at least 95%. Furthermore, the polypeptides encoded by such hybridizable polynucleotides possess the same biological function and activity as the mature polypeptides.

The full-length nucleotide sequences of the antibodies of the present invention, or fragments thereof, can generally be obtained by PCR amplification, recombinant methods, or artificial synthesis. A feasible approach is to synthesize the relevant sequences via artificial synthesis, especially when the fragment length is relatively short. Generally, long sequences can be obtained by first synthesizing multiple small fragments and then ligating them together. In addition, the coding sequence of the heavy chain can be fused with an expression tag (e.g., 6His) to generate a fusion protein.

Once the relevant sequence is obtained, large-scale production of the sequence can be achieved via recombinant methods. This usually involves cloning the sequence into a vector, transforming the vector into a host cell, and then isolating the relevant sequence from the propagated host cells using conventional methods. The biomolecules (nucleic acids, proteins, etc.) involved in the present invention include those existing in an isolated form.

Currently, DNA sequences encoding the proteins of the present invention (or fragments or derivatives thereof) can be completely synthesized by chemical synthesis. Such DNA sequences can then be introduced into various existing DNA molecules (or e.g., vectors) and cells known in the art. In addition, mutations can be introduced into the protein sequences of the present invention through chemical synthesis.

The present invention also relates to vectors comprising the appropriate DNA sequences described above, together with suitable promoters or regulatory sequences. These vectors can be used to transform suitable host cells to enable the expression of proteins.

Host cells may be prokaryotic cells, such as bacterial cells; lower eukaryotic cells, such as yeast cells; or higher eukaryotic cells, such as mammalian cells. Representative examples include: bacterial cells such as *Escherichia coli, Streptomyces* spp., and *Salmonella typhimurium;* fungal cells such as yeast; insect cells such as *Drosophila* S2 or Sf9 cells; and animal cells such as CHO, COS7, and 293 cells, etc.

Transformation of host cells with recombinant DNA can be performed using conventional techniques well known to those skilled in the art. When the host is a prokaryote (e.g., *Escherichia coli*), competent cells capable of DNA uptake can be harvested after the exponential growth phase and treated by the CaCl₂ method, the steps of which are well established in the art. An alternative approach involves the use of MgCl₂. If necessary, transformation can also be carried out via electroporation. When the host is a eukaryote, the following DNA transfection methods can be employed: calcium phosphate co-precipitation, conventional mechanical methods such as microinjection, electroporation, liposome encapsulation, etc.

The obtained transformants can be cultured by conventional methods to express the polypeptides encoded by the genes of the present invention. Depending on the host cells employed, the culture medium used for cultivation can be selected from various conventional culture media. Cultivation is performed under conditions suitable for the growth of the host cells. After the host cells have grown to an appropriate cell density, the selected promoter is induced by a suitable method (e.g., temperature shift or chemical induction), followed by an additional period of cell culture.

The recombinant polypeptides produced by the aforementioned methods can be expressed intracellularly, on the cell membrane, or secreted extracellularly. If necessary, the recombinant proteins can be isolated and purified by various separation methods utilizing their physical, chemical, and other properties. These methods are well known to those skilled in the art, including but not limited to: conventional refolding treatment, treatment with protein precipitants (salting-out method), centrifugation, osmotic lysis, ultrafiltration, ultracentrifugation, molecular sieve chromatography (gel filtration), adsorption chromatography, ion exchange chromatography, high-performance liquid chromatography (HPLC), various other liquid chromatography techniques, and combinations thereof.

The single-domain antibodies or antibodies of the present invention can be used alone, or conjugated or coupled to detectable labels (for diagnostic purposes), therapeutic agents, protein kinase (PK) modification moieties, or any combinations of the foregoing substances.

Detectable labels for diagnostic use include but are not limited to: fluorescent or luminescent labels, radioactive labels, MRI (magnetic resonance imaging) or CT (computed tomography) contrast agents, or enzymes capable of generating detectable products.

Therapeutic agents that can be conjugated or coupled to the antibodies of the present invention include but are not limited to: 1. radionuclides; 2. biotoxins; 3. cytokines such as IL-2, etc.; 4. gold nanoparticles/nanorods; 5. viral particles; 6. liposomes; 7. nanomagnetic particles; 8. prodrug-activating enzymes (e.g., DT-diaphorase (DTD) or biphenyl hydrolase-like protein (BPHL)), etc.

### HER2 protein

Human epidermal growth factor receptor 2 (HER2, ERBB2) is a tyrosine kinase receptor membrane glycoprotein encoded by the *ErbB* gene, and belongs to the epidermal growth factor receptor family. In humans, this gene is located on chromosome 17q21 and is classified as a proto-oncogene. Its encoded product, the HER2 protein, is a 185 kDa transmembrane glycoprotein (abbreviated as p185), consisting of 1255 amino acids, with residues 720-987 constituting the tyrosine kinase domain. The HER2 protein structure consists of three parts: the extracellular domain (ECD), a transmembrane domain (TM), and an intracellular domain (ICD). The extracellular domain can be divided into four subdomains (I-IV): subdomains I and III serve as ligand-binding sites, while subdomains II and IV are rich in cysteine residues, which enable the formation of homodimers or heterodimers. The transmembrane domain adopts an α-helical structure. The intracellular domain contains multiple important loop structures that form the active sites of tyrosine kinase.

The HER2 protein primarily binds to its respective ligands by forming heterodimers with other members of its family, including HER1 (EGFR), HER3, and HER4. HER2 protein is often the preferred partner for heterodimer formation, and its activity is generally stronger than that of other heterodimers. Upon binding to ligands, HER2 mainly triggers receptor dimerization and autophosphorylation of the cytoplasmic tyrosine kinase domain, thereby activating tyrosine kinase activity. The main signal transduction pathways mediated by the HER2 protein include the RAS/RAF/mitogen-activated protein kinase (MAPK) pathway, phosphatidylinositol 3-kinase (PI3K)/AKT pathway, signal transducer and activator of transcription (STAT) pathway, and phospholipase C (PLC) pathway, etc. Variant forms of HER2 include overexpression, mutation, and amplification.

By targeting the overexpressed HER2 protein, the anti-HER2 single-domain antibodies of the present invention can be used for the targeted therapy of diseases with high HER2 expression, including but not limited to: breast cancer, gastric cancer, colorectal cancer, ovarian cancer, lung cancer, prostate cancer, liver cancer, renal tumor, small intestinal cancer, large intestinal cancer, cholangiocarcinoma, cervical cancer, lymphoma, esophageal cancer, or combinations thereof.

### Anti-HER2 single-domain antibody

In the present invention, the anti-HER2 single-domain antibodies include monomers, bivalent forms (bivalent antibodies), tetravalent forms (tetravalent antibodies), and/or multivalent forms (multivalent antibodies).

In a preferred embodiment of the present invention, the amino acid sequence of the VHH chain of the anti-HER2 single-domain antibody is selected from one or more of SEQ ID NOs: 6-20.

### Labeled antibody

In a preferred embodiment of the present invention, the single-domain antibodies or antibodies may be conjugated with a detectable label. More preferably, the label is selected from the group consisting of isotopes, colloidal gold labels, chromogenic labels, and fluorescent labels.

Colloidal gold labeling can be performed using methods known to those skilled in the art. In a preferred embodiment of the present invention, the anti-HER2 single-domain antibodies or antibodies are labeled with colloidal gold to obtain colloidal gold-labeled antibodies. The anti-HER2 single-domain antibodies or antibodies of the present invention can effectively bind to the HER2 protein.

### Detection method

The present invention further relates to a method for detecting the HER2 protein or fragments thereof. The method generally comprises the following steps: obtaining cell and/or tissue samples; lysing the samples in a medium; detecting the level of the HER2 protein in the lysed samples.

In the detection method of the present invention, there are no particular limitations on the samples used. A representative example is a cell-containing sample preserved in cell preservation solution.

### Kit

The present invention further provides a kit comprising the anti-HER2 single-domain antibodies or antibodies (or fragments thereof) of the present invention, or a detection plate. In a preferred embodiment of the present invention, the kit further comprises containers, instructions for use, buffers, and the like.

The present invention further provides a detection kit for determining the level of HER2 protein. The kit comprises antibodies that recognize the HER2 protein, a lysis medium for lysing samples, general reagents and buffers required for detection, such as various buffers, detection labels, detection substrates, and the like. The detection kit may be an *in vitro* diagnostic device.

### Pharmaceutical composition

The present invention further provides a pharmaceutical composition. It comprises the anti-HER2 single-domain antibody of the first aspect of the present invention, the anti-HER2 antibody of the second aspect of the present invention, the chimeric antigen receptor of the third aspect of the present invention, the fusion protein of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof, as an active ingredient, and a pharmaceutically acceptable carrier.

Typically, the aforementioned substances can be formulated in a non-toxic, inert, pharmaceutically acceptable aqueous carrier medium, wherein the pH is usually about 5-8, preferably about 6-8, although the pH may vary depending on the properties of the formulated substances and the condition to be treated. The formulated pharmaceutical composition can be administered via conventional routes, including but not limited to: intraperitoneal, intravenous, or topical administration.

The pharmaceutical composition of the present invention contains a safe and effective amount (e.g., 0.001-99 wt%, preferably 0.01-90 wt%, more preferably 0.1-80 wt%) of the aforementioned antibodies (or conjugates thereof) of the present invention, as well as pharmaceutically acceptable carriers or excipients. Such carriers include (but are not limited to): saline, buffers, glucose, water, glycerol, ethanol, and combinations thereof. The pharmaceutical preparation shall be compatible with the route of administration. The pharmaceutical composition of the present invention can be formulated into injectable formulations, for example, prepared by conventional methods using normal saline or aqueous solutions containing glucose and other adjuvants. Pharmaceutical compositions such as injections and solutions should be manufactured under sterile conditions. The administered dose of the active ingredient is a therapeutically effective amount, e.g., about 10 µg/kg body weight to about 50 mg/kg body weight per day. In addition, the polypeptides of the present invention can also be used in combination with other therapeutic agents.

In the administration of the pharmaceutical composition, a safe and effective amount of the immunoconjugate is administered to a mammal, wherein the safe and effective amount is usually at least about 10 µg/kg body weight, and in most cases does not exceed about 50 mg/kg body weight, preferably the dose ranges from about 10 µg/kg body weight to about 10 mg/kg body weight. Of course, the specific dose should also take into account factors such as the route of administration and the patient's health status, which are within the skill scope of a skilled physician.

### Application

As described above, the single-domain antibodies and antibodies of the present invention have broad biological and clinical application value, with their applications spanning multiple fields including the diagnosis and treatment of HER2 proteinassociated diseases, basic medical research, and biological research. A preferred application is for the clinical diagnosis, prevention, and treatment of diseases targeting the HER2 protein.

The present invention also provides a method for stimulating T cell-mediated immune responses targeting tumor cell populations or tissues in mammals, comprising the following step: administering the CAR-T cells of the present invention to a mammal.

In one embodiment, the present invention encompasses a type of cellular therapy, which involves isolating autologous T cells from a patient (or allogeneic donors), activating and genetically engineering the cells to generate CAR-T cells, followed by infusing the cells back into the same patient. This approach minimizes the risk of graft-versus-host reaction, and antigens are recognized by T cells in an MHC-unrestricted manner. Furthermore, a single type of CAR-T cells can treat all cancers expressing the target antigen. Unlike antibody therapy, CAR-T cells are capable of *in vivo* proliferation, resulting in long-term persistence that can achieve sustained tumor control.

In one embodiment, the CAR-T cells of the present invention can undergo stable *in vivo* expansion and persist for several months to years. Additionally, the CAR-mediated immune response can be part of an adoptive immunotherapy regimen, wherein the CAR-T cells induce a specific immune response against tumor cells with high expression of the antigen recognized by the CAR antigen-binding domain. For example, the CAR-T cells of the present invention elicit a specific immune response against tumor cells with high HER2 expression.

Treatable cancers include non-vascularized or substantially non-vascularized tumors, as well as vascularized tumors. Cancer types treated with the CARs of the present invention include but are not limited to: breast cancer, gastric cancer, colorectal cancer, ovarian cancer, lung cancer, prostate cancer, liver cancer, renal tumor, small intestinal cancer, large intestinal cancer, cholangiocarcinoma, cervical cancer, lymphoma, esophageal cancer, etc.

Typically, the activated and expanded cells as described herein can be used for the treatment and prevention of diseases such as tumors. Therefore, the present invention provides a method for treating cancer, comprising administering a therapeutically effective amount of the CAR-T cells of the present invention to a subject in need thereof.

The CAR-T cells of the present invention can be administered alone or as a pharmaceutical composition in combination with diluents and/or other components such as IL-2, IL-17, or other cytokines or cell populations. Briefly, the pharmaceutical composition of the present invention may comprise the target cell population as described herein, in combination with one or more pharmaceutically or physiologically acceptable carriers, diluents, or excipients.

The pharmaceutical composition of the present invention can be administered in a manner suitable for the disease to be treated (or prevented). The dosage and frequency of administration will be determined by factors such as the patient's condition and the type and severity of the patient's disease, or can be established through clinical trials.

When referring to an "immunologically effective amount", "anti-tumor effective amount", "tumor-suppressive effective amount", or "therapeutic amount", the precise quantity of the composition of the present invention to be administered can be determined by a physician, taking into account the patient's (subject's) age, weight, tumor size, degree of infection or metastasis, and individual differences in the patient's condition. Pharmaceutical compositions comprising the T cells described herein can be administered at a dose of 10⁴ to 10⁹ cells/kg body weight, preferably 10⁵ to 10⁷ cells/kg body weight (including all integer values within this range). The T cell composition can also be administered multiple times at these doses. Cells can be administered using injection techniques well known in immunotherapy (see, e.g., Rosenberg et al., New Eng. J. of Med. 319:1676, 1988). The optimal dosage and treatment regimen for a specific patient can be readily determined by those skilled in the medical field by monitoring the disease signs of the patient, thus adjusting the treatment accordingly.

Administration of the composition to a subject can be performed by any convenient means, including nebulization, injection, ingestion, infusion, implantation, or transplantation. The compositions described herein can be administered to a patient subcutaneously, intradermally, intratumorally, intranodally, intraspinally, intramuscularly, via intravenous injection, or intraperitoneally. In one embodiment, the T cell composition of the present invention is administered to a patient via intradermal or subcutaneous injection. In another embodiment, the T cell composition of the present invention is preferably administered via intravenous injection. The T cell composition can be directly injected into tumors, lymph nodes, or sites of infection.

In certain embodiments of the present invention, cells activated and expanded using the methods described herein or other methods known in the art for expanding T cells to therapeutic levels are administered to a patient in combination with (e.g., before, concurrently with, or after) any number of relevant treatment modalities, including but not limited to treatment with agents such as antiviral therapy, cidofovir and interleukin-2, cytarabine (also known as ARA-C), or natalizumab for patients with multiple sclerosis (MS), efalizumab for patients with psoriasis, or other treatments for patients with progressive multifocal leukoencephalopathy (PML). In further embodiments, the T cells of the present invention can be used in combination with chemotherapy, radiation therapy, immunosuppressants such as cyclosporine, azathioprine, methotrexate, mycophenolate mofetil, and FK506, antibodies, or other immunotherapeutic agents. In further embodiments, the cell composition of the present invention is administered to a patient in combination with (e.g., before, concurrently with, or after) bone marrow transplantation, or treatment with chemotherapeutic agents such as fludarabine, external beam radiation therapy (XRT), or cyclophosphamide. For example, in one embodiment, a subject may undergo standard treatment with high-dose chemotherapy followed by peripheral blood stem cell transplantation. In some embodiments, following transplantation, the subject receives an infusion of the expanded immune cells of the present invention. In an additional embodiment, the expanded cells are administered before or after surgery.

The dosage of the above treatments administered to a patient will vary depending on the precise nature of the condition being treated and the recipient of the treatment. Dosage ratios for human administration can be implemented in accordance with practices accepted in the art. Typically, 1×10⁵ to 1×10¹⁰ modified T cells of the present invention can be administered to a patient per treatment or per course of treatment, e.g., by intravenous reinfusion.

### Main advantages of the present invention

1. The anti-HER2 single-domain antibodies of the present invention are characterized by high affinity, simple structure, and ease of preparation, and they bind to different antigenic epitopes from those targeted by Trastuzumab and Pertuzumab, thus holding broad application prospects in fields such as tumor therapy and immunoassay.
2. The present invention also prepares fusion proteins by conjugating the constructed anti-HER2 single-domain antibodies with cytokines (e.g., IL-12 protein). Said fusion proteins can not only target tumor lesions, but also accurately release cytokines and stimulate immune cells to exert immune killing functions. The aforementioned fusion proteins are capable of locally and precisely increasing the effective concentration of cytokines in the tumor microenvironment, thereby avoiding systemic toxic reactions.

The present invention will be further illustrated below with reference to the specific examples. It should be understood that these examples are only to illustrate the present invention, not to limit the scope of the present invention. The conditions of the experimental methods not specifically indicated in the following examples are usually in accordance with conventional conditions as described in e.g., Sambrook et al., Molecular Cloning: A Laboratory Manual (New York: Cold Spring Harbor Laboratory Press, 1989), or according to the conditions recommended by the manufacturers. Percentages and parts are calculated by weight unless otherwise stated.

### Sequence listing

| Sequence Number (SEQ ID NO) | Sequence | Sequence Information |
|---|---|---|
| 1 | TQVCTGTDMKLRLPASPETHLDMLRHLYQGCQVVQGNL ELTYLPTNASLSFLQDIQEVQGYVLIAHNQVRQVPLQRLR IVRGTQLFEDNYALAVLDNGDPLNNTTPVTGASPGGLRE LQLRSLTEILKGGVLIQRNPQLCYQDTILWKDIFHKNNQL ALTLIDTNRSRACHPCSPMCKGSRCWGESSEDCQSLTRTV CAGGCARCKGPLPTDCCHEQCAAGCTGPKHSDCLACLH FNHSGICELHCPALVTYNTDTFESMPNPEGRYTFGASCVT ACPYNYLSTDVGSCTLVCPLHNQEVTAEDGTQRCEKCSK PCARVCYGLGMEHLREVRAVTSANIQEFAGCKKIFGSLA FLPESFDGDPASNTAPLQPEQLQVFETLEEITGYLYISAWP DSLPDLSVFQNLQVIRGRILHNGAYSLTLQGLGISWLGLR SLRELGSGLALIHHNTHLCFVHTVPWDQLFRNPHQALLH TANRPEDECVGEGLACHQLCARGHCWGPGPTQCVNCSQ FLRGQECVEECRVLQGLPREYVNARHCLPCHPECQPQNG SVTCFGPEADQCVACAHYKDPPFCVARCPSGVKPDLSYM PIWKFPDEEGACQPCPINCTHSCVDLDDKGCPAEQRASPL T | Amino acid sequence of the HER2-His recombinant protein |
| 2 | GTCCTGGCTGCTCTTCTACAAGG | Forward primer F1 |
| 3 | GGTACGTGCTGTTGAACTGTTCC | Reverse primer R1 |
| 4 | ATGGCCCAGGTGCAGCTGCAGGAGTCTGGRGGAGG | Forward primer F2 |
| 5 | GTGGTGTGAGGAGACGGTGACCTGGGT | Reverse primer R2 |
| 6 | QVQLQESGGGLVQAEGSLRLSCAASGRTFSTYDIGWFRQ APGKEREFVARITRYGTGPLYAGSVKGRFTISRDNAKNTV YLQMNGLKPEDTAVYYCAVQSGIGRVYDYRASVSYTYW GQGTQVTVSS | HER2-1 VHH |
| 7 | QVQLQESGGGLVQAGDSLRLSCAASGLTFSTVGMGWFR QLLGKEREPVAAISWTGNVIGYGDSVKGRFTISRDSAKNT VYLQMNSLKPEDTAVYYCAARRRGTSSYDYWGQGTQV TVSS | HER2-2 VHH |
| 8 | QVQLQESGGGLVQAGDSLRLSCAASGLTFSTVGMGWFR QVLGMEREPVAGISWTGNVIGYAESVTGRFTISRDSAKNT VYLQMNSLKPEDTAVYYCAARRRGMSSYDYWGQGTQV TVSS | HER2-3 VHH |
| 9 | QVQLQESGGGLVPAEGSLRLSCAASGRTFIPYDIGWFRQA PGKEREFVAHINRLGIGALYAGSVEGRVTISRDNVKNTVY LQMNGLKPEDTAVYYCAAQSGIGAIYDYRASVSYTYWG QGTQVTVSS | HER2-4 VHH |
| 10 | QVQLQESGGGLVQAGDSLRLSCAASERTFARYVMGWFR QAPGKDREFVAHIYSSGSTAYEGSVKGRFTISRDNAKNTV YLQMNSLKPEDTAVYYCAARDRDSTWNQGTSHWGQGT QVTVSS | HER2-5 VHH |
| 11 | QVQLQESGGGLVQAGGSLRLSCAASGRTFSGAGVGWFR QDPEKEREFVAAIAWSGGSTRYADSVKGRFTISRDNTENT VFLQMNNLRPEDTAVYYCAATRRFYSGLTYTQRDVYDN WGQGTQVTVSS | HER2-8 VHH |
| 12 | QVQLQESGGGLVQPGGSLRLSCTAARNIFGRNVMGWFR QAPGKQREFVAHITGGSAVIYATSVKGRFTISRDNAKNTV YLQMNNLTLDDTAVYFCRAVNDDLWGQGTQVTVSS | HER2-10 VHH |
| 13 | QVQLQESGGGLVQAGGSLRLSCAASGHTVSRNMMGWF RQAPGKEREFVSAIAWNGEDTYYAESVEGRFTISKDNVK NTIYLQMNSLKPEDTAVYYCAASLFRLWNSATAGNNRV YHYWGQGTQVTVSS | HER2-11 VHH |
| 14 | QVQLQESGGGLVQPGGSLRLSCAPSGSIFAFNAMGWYRQ APGKQRELVATITKEGNTNYVDSVKGRFTISRDNYKNTV DLHMTSLKPDDTAVYYCNARDTRKWVSGGYDYWGQGT QVTVSS | HER2-16 VHH |
| 15 | QVQLQESGGGLVQAGGSLRLTCAASGGTISTDVMGWFR QDPGKEREFVAAIQWSRDYTYYSDSVKGRFTGSRDNAK NTVYLQMNSLKPEDAAVYYCARHWSGDIYASNSYNSW GQGTQVTVSS | HER2-32 VHH |
| 16 | QVQLQESGGGLVQPGGSLNLSCAASRSIFNALMGWYRQ APGNQREFVAHITRGGSTMYADSVKGRFTISRDNAKNTV YLQMNGLKPDDSAVYYCRDWGHDSWGQGTQVTVSS | HER2-34 VHH |
| 17 | QVQLQESGGGLVQAGGSLRLSCAASGRTFSSYDVAWFR QAPEKEREFVAGIRWRGSIAYYVDSVKGRFTISRDNAKN TVYLQMNGLKPEDTAVYYCAATVRTYYGENYDRDASA YGYWGQGTQVTVSS | HER2-37 VHH |
| 18 | QLQESGGGLVQAGDSLRLSCAASERTFARYVMGWFRQA PGKDREFVAHIYSSGSTAYEGSVKGRFTISRDNAKNTVYL QMNNLKPEDTAVYYCAARPRDTVWTSSASWGQGTQVT VSS | HER2-38 VHH |
| 19 | RVQLQESGGGLVQAGGSMRLSCAASGLTFSTVGMGWFR QLLGKEREPVAAISWTGNVIGYGDSVKGRFTISRDSAKNT VYLQMNSLKPEDTAVYYCAARRRGTSSYDYWGQGTQV TVSS | HER2-40 VHH |
| 20 | QVQLQESGGGLVQAGGSLRLSCAASGRIESSYVVGWFRQ PPGKEREFVTSIPWSGGATAYAGSVKGRFTISRDNAKNTL YLQMNNLKPEDTAVYYCAARTRDSVWTSSTSWGQGTQ VTVSS | HER2-41 VHH |
| 21 | CCTTAAGGGCGTGCAGTGCCAGGTGCAGCTGCAGGAG TC | Primer HF |
| 22 | GATTTGGGCTCGCTAGCTGAGGAGACGGTGACCTGGG | Primer HR |
| 23 | DIQMTQSPSSLSASVGDRVTITCRASQDVNTAVAWYQQK PGKAPKLLIYSASFLYSGVPSRFSGSRSGTDFTLTISSLQPE DFATYYCQQHYTTPPTFGQGTKVEIKRTVAAPSVFIFPPS DEQLKSGTASVVCLLNNFYPREAKVQWKVDNALQSGNS QESVTEQDSKDSTYSLSSTLTLSKADYEKHKVYACEVTH QGLSSPVTKSFNRGEC | Trastuzumab light chain |
| 24 | EVQLVESGGGLVQPGGSLRLSCAASGFNIKDTYIHWVRQ APGKGLEWVARIYPTNGYTRYADSVKGRFTISADTSKNT AYLQMNSLRAEDTAVYYCSRWGGDGFYAMDYWGQGT LVTVSSASTKGPSVFPLAPSSKSTSGGTAALGCLVKDYFP EPVTVSWNSGALTSGVHTFPAVLQSSGLYSLSSVVTVPSS SLGTQTYICNVNHKPSNTKVDKKVEPKSCDKTHTCPPCP APELLGGPSVFLFPPKPKDTLMISRTPEVTCVVVDVSHED PEVKFNWYVDGVEVHNAKTKPREEQYNSTYRVVSVLTV LHQDWLNGKEYKCKVSNKALPAPIEKTISKAKGQPREPQ VYTLPPSREEMTKNQVSLTCLVKGFYPSDIAVEWESNGQ PENNYKTTPPVLDSDGSFFLYSKLTVDKSRWQQGNVFSC SVMHEALHNHYTQKSLSLSPGK | Trastuzumab heavy chain |
| 25 | DIQMTQSPSSLSASVGDRVTITCKASQDVSIGVAWYQQKP GKAPKLLIYSASYRYTGVPSRFSGSGSGTDFTLTISSLQPE DFATYYCQQYYIYPYTFGQGTKVEIKRTVAAPSVFIFPPS DEQLKSGTASVVCLLNNFYPREAKVQWKVDNALQSGNS QESVTEQDSKDSTYSLSSTLTLSKADYEKHKVYACEVTH QGLSSPVTKSFNRGEC | Pertuzumab light chain |
| 26 | EVQLVESGGGLVQPGGSLRLSCAASGFTFTDYTMDWVR QAPGKGLEWVADVNPNSGGSIYNQRFKGRFTLSVDRSKN TLYLQMNSLRAEDTAVYYCARNLGPSFYFDYWGQGTLV TVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAP ELLGGPSVFLFPPKPKDTLMISRTPEVTCVVVDVSHEDPE VKFNWYVDGVEVHNAKTKPREEQYNSTYRVVSVLTVLH QDWLNGKEYKCKVSNKALPAPIEKTISKAKGQPREPQVY TLPPSREEMTKNQVSLTCLVKGFYPSDIAVEWESNGQPEN NYKTTPPVLDSDGSFFLYSKLTVDKSRWQQGNVFSCSVM HEALHNHYTQKSLSLSPGK | Pertuzumab heavy chain |
| 27 | GLTFSTVG | CDR1 of HER2-2, HER2-3, and HER2-40 |
| 28 | ISWTGNVI | CDR2 of HER2-2, HER2-3, and HER2-40 |
| 29 | AARRRGTSSYDY | CDR3 of HER2-2 and HER2-40 |
| 30 | QVQLQESGGGLVQAGDSLRLSCAAS | FR1 of HER2-2, HER2-3, and HER2-5 |
| 31 | MGWFRQLLGKEREPVAA | FR2 of HER2-2 and HER2-40 |
| 32 | GYGDSVKGRFTISRDSAKNTVYLQMNSLKPEDTAVYYC | FR3 of HER2-2 and HER2-40 |
| 33 | WGQGTQVTVSS | FR4 of the 15 single-domain antibodies of the present invention |
| 34 | GRTFSGAG | CDR1 of HER2-8 |
| 35 | IAWSGGST | CDR2 of HER2-8 |
| 36 | AATRRFYSGLTYTQRDVYDN | CDR3 of HER2-8 |
| 37 | QVQLQESGGGLVQAGGSLRLSCAAS | FR1 of HER2-8, HER2-11, HER2-37, and HER2-41 |
| 38 | VGWFRQDPEKEREFVAA | FR2 of HER2-8 |
| 39 | RYADSVKGRFTISRDNTENTVFLQMNNLRPEDTAVYYC | FR3 of HER2-8 |
| 40 | RNIFGRNV | CDR1 of HER2-10 |
| 41 | ITGGSAV | CDR2 of HER2-10 |
| 42 | RAVNDDL | CDR3 of HER2-10 |
| 43 | QVQLQESGGGLVQPGGSLRLSCTAA | FR1 of HER2-10 |
| 44 | MGWFRQAPGKQREFVAH | FR2 of HER2-10 |
| 45 | IYATSVKGRFTISRDNAKNTVYLQMNNLTLDDTAVYFC | FR3 of HER2-10 |
| 46 | GHTVSRNM | CDR1 of HER2-11 |
| 47 | IAWNGEDT | CDR2 of HER2-11 |
| 48 | AASLFRLWNSATAGNNRVYHY | CDR3 of HER2-11 |
| 49 | MGWFRQAPGKEREFVSA | FR2 of HER2-11 |
| 50 | YYAESVEGRFTISKDNVKNTIYLQMNSLKPEDTAVYYC | FR3 of HER2-11 |
| 51 | RVQLQESGGGLVQAGGSMRLSCAAS | FR1 of HER2-40 |
| 52 | GRTFSSYD | CDR1 of HER2-37 |
| 53 | IRWRGSIA | CDR2 of HER2-37 |
| 54 | AATVRTYYGENYDRDASAYGY | CDR3 of HER2-37 |
| 55 | VAWFRQAPEKEREFVAG | FR2 of HER2-37 |
| 56 | YYVDSVKGRFTISRDNAKNTVYLQMNGLKPEDTAVYYC | FR3 of HER2-37 |
| 57 | ERTFARYV | CDR1 of HER2-38 and HER2-5 |
| 58 | IYSSGST | CDR2 of HER2-38 and HER2-5 |
| 59 | AARPRDTVWTSSAS | CDR3 of HER2-38 |
| 60 | QLQESGGGLVQAGDSLRLSCAAS | FR1 of HER2-38 |
| 61 | MGWFRQAPGKDREFVAH | FR2 of HER2-38 and HER2-5 |
| 62 | AYEGSVKGRFTISRDNAKNTVYLQMNNLKPEDTAVYYC | FR3 of HER2-38 |
| 63 | GSIFAFNA | CDR1 of HER2-16 |
| 64 | ITKEGNT | CDR2 of HER2-16 |
| 65 | NARDTRKWVSGGYDY | CDR3 of HER2-16 |
| 66 | QVQLQESGGGLVQPGGSLRLSCAPS | FR1 of HER2-16 |
| 67 | MGWYRQAPGKQRELVAT | FR2 of HER2-16 |
| 68 | NYVDSVKGRFTISRDNYKNTVDLHMTSLKPDDTAVYYC | FR3 of HER2-16 |
| 69 | GRIESSYV | CDR1 of HER2-41 |
| 70 | IPWSGGAT | CDR2 of HER2-41 |
| 71 | AARTRDSVWTSSTS | CDR3 of HER2-41 |
| 72 | VGWFRQPPGKEREFVTS | FR2 of HER2-41 |
| 73 | AYAGSVKGRFTISRDNAKNTLYLQMNNLKPEDTAVYYC | FR3 of HER2-41 |
| 74 | GRTFSTYD | CDR1 of HER2-1 |
| 75 | ITRYGTGP | CDR2 of HER2-1 |
| 76 | AVQSGIGRVYDYRASVSYTY | CDR3 of HER2-1 |
| 77 | QVQLQESGGGLVQAEGSLRLSCAAS | FR1 of HER2-1 |
| 78 | IGWFRQAPGKEREFVAR | FR2 of HER2-1 |
| 79 | LYAGSVKGRFTISRDNAKNTVYLQMNGLKPEDTAVYYC | FR3 of HER2-1 |
| 80 | AARRRGMSSYDY | CDR3 of HER2-3 |
| 81 | MGWFRQVLGMEREPVAG | FR2 of HER2-3 |
| 82 | GYAESVTGRFTISRDSAKNTVYLQMNSLKPEDTAVYYC | FR3 of HER2-3 |
| 83 | GRTFIPYD | CDR1 of HER2-4 |
| 84 | INRLGIGA | CDR2 of HER2-4 |
| 85 | AAQSGIGAIYDYRASVSYTY | CDR3 of HER2-4 |
| 86 | QVQLQESGGGLVPAEGSLRLSCAAS | FR1 of HER2-4 |
| 87 | IGWFRQAPGKEREFVAH | FR2 of HER2-4 |
| 88 | LYAGSVEGRVTISRDNVKNTVYLQMNGLKPEDTAVYYC | FR3 of HER2-4 |
| 89 | AARDRDSTWNQGTSH | CDR3 of HER2-5 |
| 90 | AYEGSVKGRFTISRDNAKNTVYLQMNSLKPEDTAVYYC | FR3 of HER2-5 |
| 91 | GGTISTDV | CDR1 of HER2-32 |
| 92 | IQWSRDYT | CDR2 of HER2-32 |
| 93 | ARHWSGDIYASNSYNS | CDR3 of HER2-32 |
| 94 | QVQLQESGGGLVQAGGSLRLTCAAS | FR1 of HER2-32 |
| 95 | MGWFRQDPGKEREFVAA | FR2 of HER2-32 |
| 96 | YYSDSVKGRFTGSRDNAKNTVYLQMNSLKPEDAAVYYC | FR3 of HER2-32 |
| 97 | RSIFNALM | CDR1 of HER2-34 |
| 98 | TRGGST | CDR2 of HER2-34 |
| 99 | RDWGHDS | CDR3 of HER2-34 |
| 100 | QVQLQESGGGLVQPGGSLNLSCAAS | FR1 of HER2-34 |
| 101 | GWYRQAPGNQREFVAHI | FR2 of HER2-34 |
| 102 | MYADSVKGRFTISRDNAKNTVYLQMNGLKPDDSAVYYC | FR3 of HER2-34 |

### Example 1: Screening of Heavy-Chain Single-Domain Antibodies Targeting HER2

### 1.1 Preparation of Immunizing Antigen

The extracellular domain (23-652) of the antigen HER2 was synthesized by gene synthesis. A 6×His tag was added to its C-terminus, and the sequence was subcloned into the eukaryotic expression vector pcDNA3.1 to construct the expression vector for the HER2 recombinant protein (HER2-His). The amino acid sequence is shown below:

TQVCTGTDMKLRLPASPETHLDMLRHLYQGCQVVQGNLELTYLPTNASLSFL
QDIQEVQGYVLIAHNQVRQVPLQRLRIVRGTQLFEDNYALAVLDNGDPLNNTTPVT
GASPGGLRELQLRSLTEILKGGVLIQRNPQLCYQDTILWKDIFHKNNQLALTLIDTN
RSRACHPCSPMCKGSRCWGESSEDCQSLTRTVCAGGCARCKGPLPTDCCHEQCAA
GCTGPKHSDCLACLHFNHSGICELHCPALVTYNTDTFESMPNPEGRYTFGASCVTA
CPYNYLSTDVGSCTLVCPLHNQEVTAEDGTQRCEKCSKPCARVCYGLGMEHLREV
RAVTSANIQEFAGCKKIFGSLAFLPESFDGDPASNTAPLQPEQLQVFETLEEITGYLYI
SAWPDSLPDLSVFQNLQVIRGRILHNGAYSLTLQGLGISWLGLRSLRELGSGLALIH
HNTHLCFVHTVPWDQLFRNPHQALLHTANRPEDECVGEGLACHQLCARGHCWGP
GPTQCVNCSQFLRGQECVEECRVLQGLPREYVNARHCLPCHPECQPQNGSVTCFGP
EADQCVACAHYKDPPFCVARCPSGVKPDLSYMPIWKFPDEEGACQPCPINCTHSCV
DLDDKGCPAEQRASPLT (SEQ ID NO: 1)

The constructed HER2-His plasmid was subjected to plasmid extraction. After confirmed by sequencing to be correct, it was transfected into well-conditioned FreeStyle^{™} 293-F cells. The transfected cells were cultured in a shake flask on a horizontal shaker at 37°C, 120 rpm and 5% CO₂ for 3-4 days. The expression supernatant was collected by centrifugation and filtered through a 0.45 µm filter membrane. Affinity purification was performed using HisTrap^{™} FF, and the target protein was eluted with 500 mM imidazole to collect the samples. Buffer exchange and concentration were carried out using ultrafiltration concentrator tubes. The protein purity was determined by SDS-PAGE, and the protein concentration was quantified using a spectrophotometer.

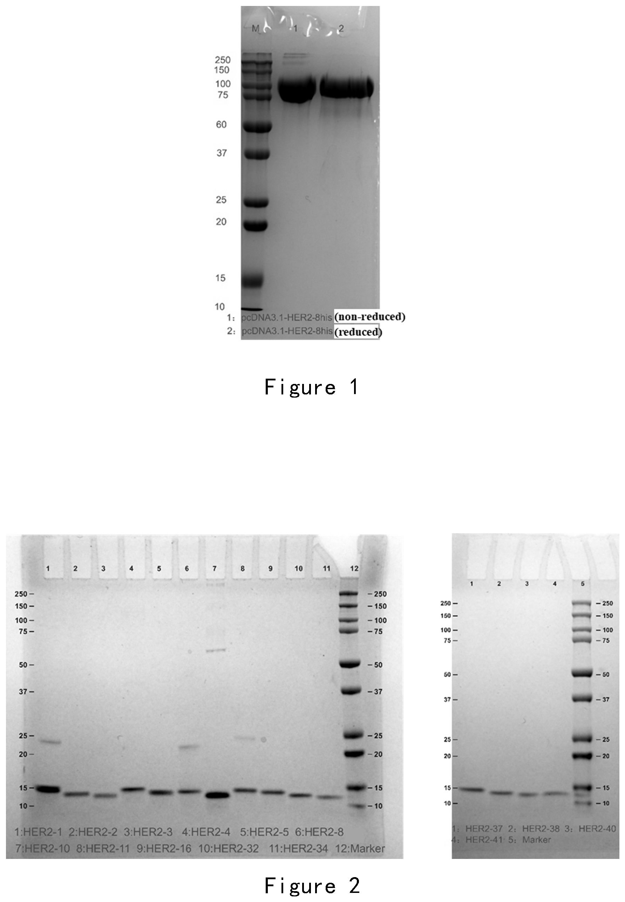

The results are shown in Figure 1. The electrophoretic position of the antigen protein was consistent with the expected value, with clear bands and high purity. No fractured or degraded bands were observed for the HER2 protein. These results indicated that the prepared purified antigen protein could be used in subsequent immunization procedures.

### 1.2 Construction of the Library

A healthy alpaca (1.5 years old, male, weighing approximately 80 kg, raised and immunized by Qingdao Kangda Biotechnology Co., Ltd.) was selected for immunization, with multiple subcutaneous injections administered at the neck and shoulder regions. After completing 5 rounds of immunization, 100 mL of peripheral blood lymphocytes were collected from the alpaca. Peripheral blood mononuclear cells (PBMCs) were isolated using the Alpaca Peripheral Blood Lymphocyte Separation Solution Kit from Tianjin Haoyang Biological Manufacture Co., Ltd. Total RNA was extracted using the RNA Keeper Tissue Stabilizer Kit from Vazyme Biotech Co., Ltd. The extracted RNA was reverse-transcribed to cDNA using the HiScript III 1st Strand cDNA Synthesis Kit (Vazyme, Cat. No. R312-01). The nucleic acid fragment encoding the variable region of the heavy-chain antibody was amplified via nested PCR:
First-round PCR:
Forward Primer F1: GTCCTGGCTGCTCTTCTACAAGG (SEQ ID NO: 2)
Reverse Primer R1: GGTACGTGCTGTTGAACTGTTCC (SEQ ID NO: 3)

The Fab fragment of the heavy-chain antibody was amplified by PCR using the obtained cDNA as the template. The reaction procedure is shown in Table 1 below:

**Table 1**

| Temperature | | | Time |
|---|---|---|---|
| 15 | 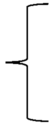 | 95°C | 5 min |
| | | 95°C | 30 s |
| | | 57°C | 30 s |
| | | 72°C | 30 s |
| | | 72°C | 7 min |

Electrophoresis was performed using a 2% agarose gel. The band of approximately 700 bp was excised, and the target gene was recovered using the Gel Extraction Kit (Vazyme) according to the manufacturer's instructions.

Second-round PCR:
Using the products of the first-round PCR as templates, another round of PCR amplification was performed to obtain single-domain antibodies. The reaction system and procedure are shown in the table below.

Forward Primer F2:
ATGGCCCAGGTGCAGCTGCAGGAGTCTGGRGGAGG (SEQ ID NO: 4)
Reverse Primer R2: GTGGTGTGAGGAGACGGTGACCTGGGT (SEQ ID NO: 5)

The reaction system is shown in Table 2 below:

**Table 2**

| Component | Volume |
|---|---|
| First-round PCR Product | 1 µL |
| VHH-FOR (10 µM) | 1 µL |
| VHH-BACK (10 µM) | 1 µL |
| 2×PCR Bestaq^{™} Master Mix | 25 µL |
| ddH₂O | Up to 50 µL |

The PCR reaction procedure is shown in Table 3 below:

**Table 3**

| Temperature | | | Time |
|---|---|---|---|
| 20 | 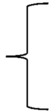 | 95°C | 5 min |
| | | 95°C | 30 s |
| | | 55°C | 30 s |
| | | 72°C | 30 s |
| | | 72°C | 5 min |

The target nucleic acid fragment of the heavy-chain single-domain antibody was recovered and cloned into the phage display vector pMES4 using restriction endonucleases PstI and BsteII (purchased from NEB). Fresh competent cells TG1 were prepared, and the ligation product was electroporated into the competent cells TG1. The transformation efficiency and accuracy of the electroporated competent cells were verified. Gradient dilution and plating were performed to calculate the library capacity, which was determined to be 10¹¹. Twenty clones were randomly selected for colony PCR and sequencing verification. The results showed that the insertion rate reached 100%, indicating the successful construction of a phage display library of heavy-chain single-domain antibodies targeting HER2.

### 1.3 Panning of Heavy-Chain Single-Domain Antibodies Targeting HER2

Immunotubes were coated with the HER2-His fusion protein at a concentration of 10 µg/mL and incubated overnight at 4 °C. On the following day, the immunotubes were blocked with 1 mL of PBS milk (PBS containing 2% skimmed milk powder) at 37 °C for 1 h, and simultaneously, 100 µL of the phage library (derived from the heavy-chain single-domain antibody phage display library constructed in Example 1.2) was subjected to blocking treatment.

The blocked phage antibody library was added to the immunotubes and incubated for binding overnight at 4 °C. The liquid in the immunotubes was discarded, and the immunotubes were washed sequentially with PBS, PBST and PBS supplemented with NaCl, with the washing stringency increased gradually with each round.

After 3 rounds of washing, the liquid in the immunotubes was discarded, and 1 mL of 0.2 mol/L glycine-hydrochloric acid (pH 2.2) was added for elution. The eluate was neutralized to pH 7.4 with 1 mol/L Tris. *Escherichia coli* TG1 cells in the logarithmic growth phase were added to the immunotubes for infection. The eluted and infected bacterial suspension was resuspended; after precipitation, the precipitate was evenly spread onto 2YT-AG (A: ampicillin sodium; G: glucose) agar plates. After static incubation overnight at 37 °C, all colonies were collected.

An appropriate volume of the bacterial suspension was inoculated into 100 mL of 2YT-AG liquid medium and cultured until the absorbance (A) value at 600 nm reached 0.7. Helper phage M13KO7 was added at a multiplicity of infection (MOI) of 50:1, followed by static incubation at room temperature for 30 min and subsequent incubation at 37 °C and 150 r/min for 1 h. Isopropyl β-D-thiogalactoside (IPTG) was added to a final concentration of 0.15 mmol/L, and the culture was incubated at 30 °C and 200 rpm for 10 h. Phage antibodies were recovered by precipitation with PEG8000, and a certain amount of the recovered phage antibodies was used for the next round of panning.

After 3 repeated rounds of panning, positive clones were finally enriched, thus achieving the goal of screening HER2-specific antibodies via phage display technology.

### 1.4 Screening of Positive Clones by Phage Enzyme-Linked Immunosorbent Assay (ELISA)

Single monoclonal colonies obtained from panning were picked and inoculated into 96-well plates containing 2×YT-AG medium, followed by culture overnight at 37 °C and 220 rpm until saturation. The saturated bacterial culture was subcultured to an OD₆₀₀ of approximately 0.5, and helper phage M13KO7 was added at a multiplicity of infection (MOI) of 50:1. After static incubation at room temperature for 30 min, the culture was then incubated at 37 °C and 150 rpm for 1 h. Kanamycin was added to a final concentration of 20 µg/mL, and IPTG was added to a final concentration of 0.15 mM, followed by induction for phage display at 30 °C and 200 rpm for 12 h.

The supernatant was collected by centrifugation as the phage antibody preparation. The target antigen (HER2-His) and control antigen (BSA) were coated onto the plates and incubated overnight at 4 °C. The phage antibody preparations were added and incubated at 37 °C for 1 h to allow binding. After washing, the anti-M13 antibody (HRP-conjugated polyclonal anti-M13 phage antibody, secondary antibody) was added and incubated for binding at 37 °C for 45 min. Following another round of washing, single-component TMB chromogenic solution was added. The absorbance was measured at a wavelength of 450 nm. Monoclonal colonies with an OD value of the sample well more than 3 times that of the control well were identified as positive clones. These positive clones were sent for sequencing to obtain the variable region genes of the candidate antibodies. The antibody sequence information is shown below:
HER2-1: 
QVQLQESGGGLVQAEGSLRLSCAASGRTFSTYDIGWFRQAPGKEREFVARITRYGTGP
LYAGSVKGRFTISRDNAKNTVYLQMNGLKPEDTAVYYCAVQSGIGRVYDYRASVSYT
YWGQGTQVTVSS (SEQ ID NO: 6)
HER2-2: QVQLQESGGGLVQAGDSLRLSCAASGLTFSTVGMGWFRQLLGKEREPVAAISWTGNV 
IGYGDSVKGRFTISRDSAKNTVYLQMNSLKPEDTAVYYCAARRRGTSSYDYWGQGTQ
VTVSS (SEQ ID NO: 7)
HER2-3: 
QVQLQESGGGLVQAGDSLRLSCAASGLTFSTVGMGWFRQVLGMEREPVAGISWTGNV
IGYAESVTGRFTISRDSAKNTVYLQMNSLKPEDTAVYYCAARRRGMSSYDYWGQGTQ
VTVSS (SEQ ID NO: 8)
HER2-4: 
QVQLQESGGGLVPAEGSLRLSCAASGRTFIPYDIGWFRQAPGKEREFVAHINRLGIGAL
YAGSVEGRVTISRDNVKNTVYLQMNGLKPEDTAVYYCAAQSGIGAIYDYRASVSYTY
WGQGTQVTVSS (SEQ ID NO: 9)
HER2-5: 
QVQLQESGGGLVQAGDSLRLSCAASERTFARYVMGWFRQAPGKDREFVAHIYSSGST
AYEGSVKGRFTISRDNAKNTVYLQMNSLKPEDTAVYYCAARDRDSTWNQGTSHWGQ
GTQVTVSS (SEQ ID NO: 10)
HER2-8: 
QVQLQESGGGLVQAGGSLRLSCAASGRTFSGAGVGWFRQDPEKEREFVAAIAWSGGS
TRYADSVKGRFTISRDNTENTVFLQMNNLRPEDTAVYYCAATRRFYSGLTYTQRDVY
DNWGQGTQVTVSS (SEQ ID NO: 11)
HER2-10: 
QVQLQESGGGLVQPGGSLRLSCTAARNIFGRNVMGWFRQAPGKQREFVAHITGGSAVI
YATSVKGRFTISRDNAKNTVYLQMNNLTLDDTAVYFCRAVNDDLWGQGTQVTVSS
(SEQ ID NO: 12)
HER2-11: 
QVQLQESGGGLVQAGGSLRLSCAASGHTVSRNMMGWFRQAPGKEREFVSAIAWNGE
DTYYAESVEGRFTISKDNVKNTIYLQMNSLKPEDTAVYYCAASLFRLWNSATAGNNR
VYHYWGQGTQVTVSS (SEQ ID NO: 13)
HER2-16: 
QVQLQESGGGLVQPGGSLRLSCAPSGSIFAFNAMGWYRQAPGKQRELVATITKEGNTN
YVDSVKGRFTISRDNYKNTVDLHMTSLKPDDTAVYYCNARDTRKWVSGGYDYWGQ
GTQVTVSS (SEQ ID NO: 14)
HER2-32: 
QVQLQESGGGLVQAGGSLRLTCAASGGTISTDVMGWFRQDPGKEREFVAAIQWSRDY
TYYSDSVKGRFTGSRDNAKNTVYLQMNSLKPEDAAVYYCARHWSGDIYASNSYNSW
GQGTQVTVSS (SEQ ID NO: 15)
HER2-34: 
QVQLQESGGGLVQPGGSLNLSCAASRSIFNALMGWYRQAPGNQREFVAHITRGGSTM
YADSVKGRFTISRDNAKNTVYLQMNGLKPDDSAVYYCRDWGHDSWGQGTQVTVSS
(SEQ ID NO: 16)
HER2-37: 
QVQLQESGGGLVQAGGSLRLSCAASGRTFSSYDVAWFRQAPEKEREFVAGIRWRGSIA
YYVDSVKGRFTISRDNAKNTVYLQMNGLKPEDTAVYYCAATVRTYYGENYDRDASA
YGYWGQGTQVTVSS (SEQ ID NO: 17)
HER2-38: 
QLQESGGGLVQAGDSLRLSCAASERTFARYVMGWFRQAPGKDREFVAHIYSSGSTAY
EGSVKGRFTISRDNAKNTVYLQMNNLKPEDTAVYYCAARPRDTVWTSSASWGQGTQ
VTVSS (SEQ ID NO: 18)
HER2-40: 
RVQLQESGGGLVQAGGSMRLSCAASGLTFSTVGMGWFRQLLGKEREPVAAISWTGNV
IGYGDSVKGRFTISRDSAKNTVYLQMNSLKPEDTAVYYCAARRRGTSSYDYWGQGTQ
VTVSS (SEQ ID NO: 19)
HER2-41:

QVQLQESGGGLVQAGGSLRLSCAASGRIESSYVVGWFRQPPGKEREFVTSIPWSGGAT
AYAGSVKGRFTISRDNAKNTLYLQMNNLKPEDTAVYYCAARTRDSVWTSSTSWGQG
TQVTVSS (SEQ ID NO: 20)

### Example 2: Evaluation and Identification of Heavy-Chain Single-Domain Antibodies Targeting HER2

### 2.1 Expression and Purification of Heavy-Chain Single-Domain Antibodies in Escherichia coli

Plasmids were extracted from the 15 single-domain antibody strains obtained by sequencing analysis and transformed into BL21 (DE3) competent cells via the heat shock method. In a clean bench, 1 mL of the positive clone bacterial culture was inoculated into 100 mL of LB liquid medium (containing 100 µg/mL ampicillin) and cultured with shaking at 37 °C until the OD₆₀₀ reached approximately 0.8. IPTG was added to a final concentration of 1 mM, followed by shaking culture overnight at 30 °C.

On the following day, the bacterial cells were harvested by centrifugation at 8000 rpm for 10 min. The pellet was resuspended in 1.5 mL of pre-chilled TES buffer and stirred in an ice bath for 30 min. Then 3.0 mL of TES/4 (TES buffer diluted 4-fold with ultrapure water) was added, and the mixture was continuously stirred in an ice bath for another 30 min. The supernatant (periplasmic extract) was collected by centrifugation at 9000 rpm and 4 °C for 10 min and subjected to SDS-PAGE analysis. Furthermore, affinity purification was performed using a nickel pre-packed column on a protein purification system, and the samples were collected by elution with 300 mM imidazole buffer. The antibody concentration was quantified using a spectrophotometer, and the purity of the purified antibody was determined by SDS-PAGE.

As shown in Figure 2, the results indicated that the molecular weights of the 15 purified single-domain antibodies expressed in *E. coli* were all consistent with the expected values, with clear bands and high purity.

### 2.2 Construction of the Antigen Recombinant Protein

An expression vector for the HER2-human Fc recombinant protein (HER2-Fc) was constructed. The *HER2* gene and human Fc gene were cloned into the pcDNA3.1 vector. After confirmation of correct sequencing, the vector was transfected into well-conditioned FreeStyle^{™} 293-F cells, which were then cultured in a shaker flask at 37 °C, 120 rpm and 5% CO₂ for 3.5 days. The supernatant was collected and purified using a Protein A column, with elution performed using a citric acid-sodium citrate buffer. Buffer exchange and concentration were carried out using ultrafiltration concentrator tubes, and the purity of the HER2-Fc recombinant protein was determined by SDS-PAGE.

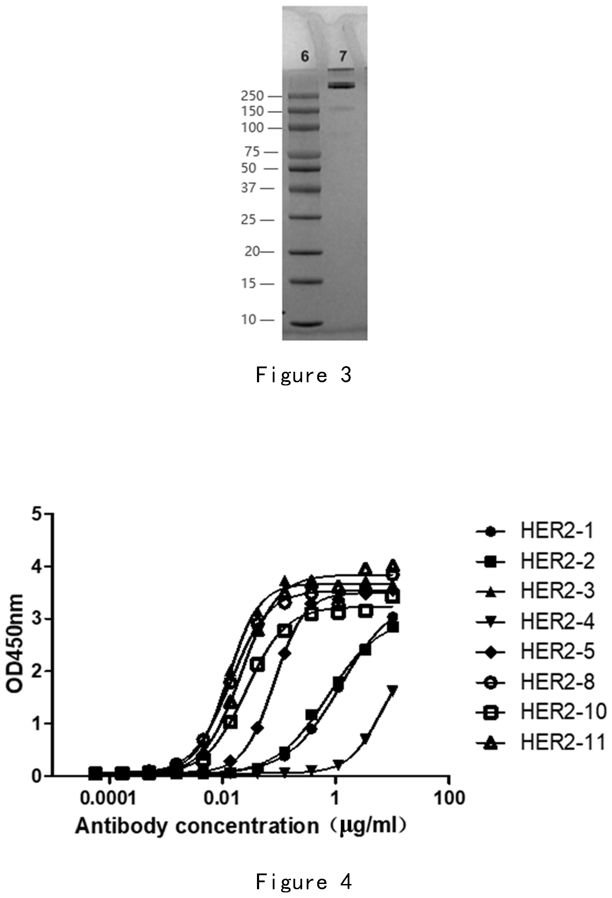

As shown in Figure 3, the results indicated that the electrophoretic position of the HER2-Fc recombinant protein was consistent with the expected value, with clear bands and high purity. No fragmentation or degradation bands were observed for the HER2-Fc recombinant protein.

Furthermore, the concentration of the HER2-Fc recombinant protein was quantified using a spectrophotometer, with a final concentration of 2 mg/mL.

### 2.3 Detection of the Binding of Candidate Anti-HER2 Heavy-Chain Single-Domain Antibodies to Human HER2 Protein

ELISA plates were coated with the human HER2-Fc fusion protein at a concentration of 200 ng per well and incubated overnight at 4 °C. Subsequently, the heavy-chain single-domain antibodies obtained in Example 2.1 were subjected to 12 serial 3-fold dilutions starting from 10 µg/mL. The plates were blocked at 37 °C for 1 h, and then the blocked whole antibodies were added at 100 µL per well, followed by static incubation at 37 °C for 1 h. After washing, HRP-conjugated anti-His tag antibody was added to the ELISA plates at 100 µL per well and incubated statically at 37 °C for 45 min. After another round of washing, TMB substrate chromogenic solution was added at 100 µL per well and incubated statically at room temperature for 10 min. The reaction was terminated by adding 100 µL of 1 M H₂SO₄ per well. The absorbance was measured at a wavelength of 450 nm using a microplate reader. Data processing and parameter fitting were performed using GraphPad Prism Software 5.0 to calculate the EC₅₀ values.

The binding activities of the 15 purified single-domain antibodies to the HER2-Fc protein were analyzed by ELISA. As shown in Figures 4-5, the results demonstrated that HER2-1, HER2-2, HER2-3, HER2-4, HER2-5, HER2-8, HER2-10, HER2-11, HER2-16, HER2-32, HER2-34, HER2-37, HER2-38, HER2-40 and HER2-41 all exhibited high binding activity to the HER2-Fc recombinant protein with typical dose-dependent characteristics.

The EC₅₀ values are shown in Tables 4 and 5 below:

**Table 4**

| Name | HER2-1 | HER2-2 | HER2-3 | HER2-4 | HER2-5 | HER2-8 | HER2-10 | HER2-11 |
|---|---|---|---|---|---|---|---|---|
| EC₅₀ | 1.213 | 0.7233 | 0.01254 | 6.519 | 0.0803 | 0.01376 | 0.02574 | 0.02075 |

**Table 5**

| Name | HER2-16 | HER2-32 | HER2-34 | HER2-37 | HER2-38 | HER2-40 | HER2-41 |
|---|---|---|---|---|---|---|---|
| EC₅₀ | 0.4201 | 1.984 | 1.24 | 1.166 | 0.02955 | 0.3275 | 0.8707 |

### 2.4 Determination of the Binding Capacity of Anti-HER2 Single-Domain Antibodies to HER2 by SPR (i.e., BIAcore) Technology

A single-concentration affinity screening assay was employed. All single-domain antibodies to be tested were assayed at the same single concentration for parallel comparison to detect the differences in binding activity among them. The capture antibody was immobilized onto the surface of CM5 chips using the Human Antibody Capture Kit, and the kinetic parameters of antibody-antigen interactions were determined via the multi-cycle kinetics approach.

Purified HER2-His was diluted to a concentration of 1 µg/mL with HBS-EP buffer, and then captured and immobilized onto the chip surface at 25 °C with a flow rate of 5 µL/min for 1 min, with the target capture response value reaching approximately 400 RU. The single-domain antibodies HER2-1, HER2-2, HER2-3, HER2-4, HER2-5, HER2-8, HER2-10, HER2-11, HER2-16, HER2-32, HER2-34, HER2-37, HER2-38, HER2-40, and HER2-41 were diluted to 1.5 µg/mL with HBS-EP buffer and used as the mobile phase. The assay conditions were set as follows: temperature at 25 °C, flow rate at 30 µL/min, association time of 60 s, and dissociation time of 120 s. The regeneration conditions were 10 mM glycine-hydrochloric acid buffer (pH 2.5) at a flow rate of 30 µL/min for 90 s. Data analysis was subsequently performed.

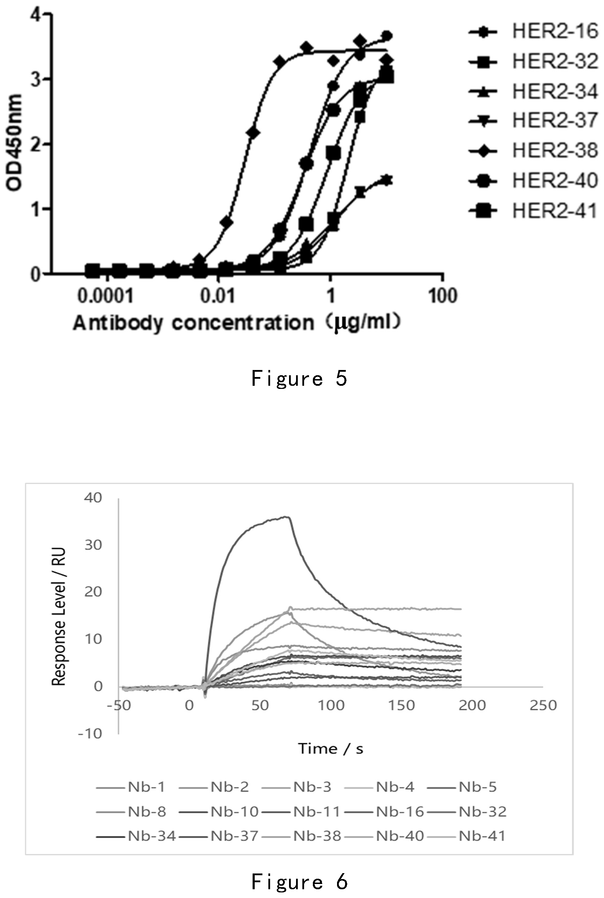

As shown in Figure 6, the association process between antibodies and antigen as well as the dissociation process of antigen-antibody complexes could be clearly and intuitively observed from the fitted curves.

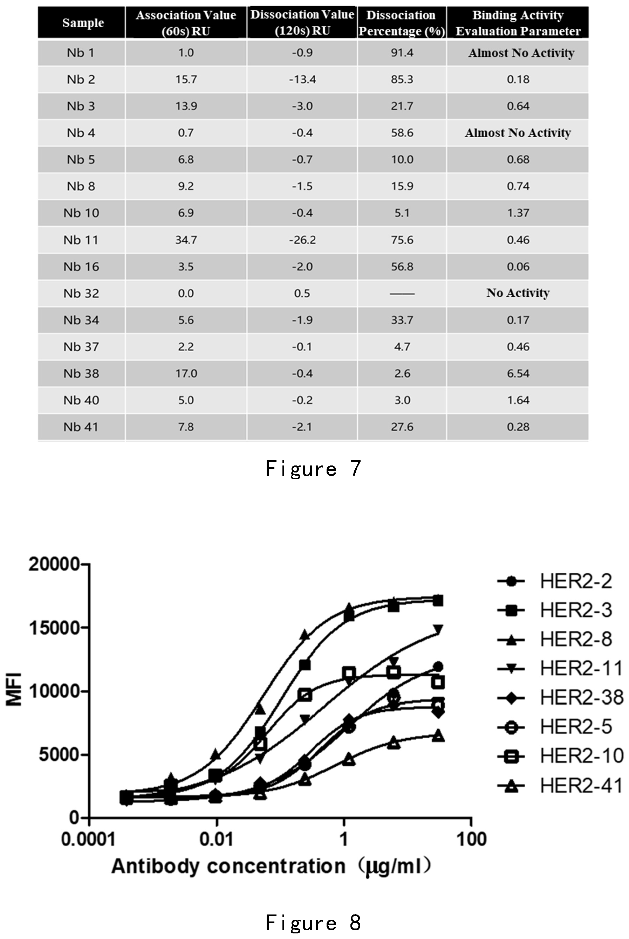

Antibody affinity is a critical parameter for evaluating antibody molecules, and surface plasmon resonance (SPR) technology is recognized as the gold standard for detecting antibody affinity. Furthermore, the inventors employed the BIAcore T100 system and the single-concentration affinity screening assay, and the binding activity parameters of HER2-1, HER2-2, HER2-3, HER2-4, HER2-5, HER2-8, HER2-10, HER2-11, HER2-16, HER2-32, HER2-34, HER2-37, HER2-38, HER2-40, and HER2-41 are presented in Figure 7.

### 2.5 Detection of Binding Activity Between Antibodies and SKBR3 Cells by FACS

SKBR3 cells were purchased (from the Cell Resource Center, Shanghai Institutes for Biological Sciences, Chinese Academy of Sciences) and cultured to the logarithmic growth phase, resuspended in FACS buffer, and adjusted to a cell density of 3×10⁶ cells/mL, with 100 µL of the cell suspension added to each well. Subsequently, the heavy-chain single-domain antibodies obtained in Example 2.1 were diluted to an initial concentration of 30 µg/mL using FACS buffer, followed by a series of 5-fold dilutions. The cell pellets were resuspended with 100 µL of the diluted primary antibody per well and incubated at 4 °C for 60 min. After washing three times with PBS, the cells were resuspended with the secondary antibody, THE^{™} His Tag Antibody [FITC], mAb, Mouse, and incubated at 4 °C for 60 min. The cells were washed three times with PBS again, then resuspended with 100-200 µL of PBS per well. The mean fluorescence intensity (MFI) was detected using a Beckman CytoFLEX flow cytometer. Data processing and parameter fitting were performed using GraphPad Prism Software 5.0 to calculate the EC₅₀ values.

As shown in Figures 8-9, the results demonstrated that HER2-3, HER2-5, HER2-8, HER2-10, HER2-11 and HER2-38 exhibited high binding activity to the HER2 protein expressed by SKBR3 cells, with typical dose-dependent characteristics.

The EC₅₀ values are shown in Tables 6 and 7 below:

**Table 6**

| Name | HER2-2 | HER2-3 | HER2-8 | HER2-11 | HER2-38 | HER2-5 | HER2-10 | HER2-41 |
|---|---|---|---|---|---|---|---|---|
| EC₅₀ | 1.103 | 0.1041 | 0.05424 | 0.4848 | 0.295 | 0.4416 | 0.06086 | 0.7466 |

**Table 7**

| Name | HER2-1 | HER2-4 | HER2-16 | HER2-32 | HER2-34 | HER2-37 | HER2-40 |
|---|---|---|---|---|---|---|---|
| EC₅₀ | 16.37 | 33.49 | 3.748 | 23.68 | 0.7497 | 2.75 | 2.119 |

### 2.6 Preparation of Anti-HER2-Fc Single-Domain Antibody Fusion Protein in Mammalian Cells

Clones identified as positive single-chain antibodies were subjected to sequencing, and the sequencing results were analyzed. The positive single-chain variable region genes were amplified using primers HF (CCTTAAGGGCGTGCAGTGCCAGGTGCAGCTGCAGGAGTC, SEQ ID NO: 21) and HR (GATTTGGGCTCGCTAGCTGAGGAGACGGTGACCTGGG, SEQ ID NO: 22). The positive single-chain antibody genes were cloned into the vector pABG1-Fc (a pABG1 vector carrying the human Fc fragment, stored in the laboratory) via the homologous recombination method.

The amplification reaction system is shown in Table 8 below:

**Table 8**

| | |
|---|---|
| Phagemid Template | 0.5 µL |
| HF Primer (10 µM) | 1 µL |
| HR Primer (10 µM) | 1 µL |
| 2×Phanta Max Master Mix | 15 µL |
| Sterilized Deionized Water | 12.5 µL |
| Total Volume | 30 µL |

The PCR amplification procedure is shown in Table 9 below:

**Table 9**

| Temperature | Time | Cycles |
|---|---|---|
| 95°C | 3min | - |
| 95°C | 15s | 30 cycles |
| 62°C | 15s | |
| 72°C | 20s | - |
| 72°C | 5min | - |
| 4°C | Hold | - |

The homologous recombination reaction system is shown in Table 10 below:

**Table 10**

| | |
|---|---|
| Vector pABG1-Fc (0.02 pmol) | 0.4 µL |
| HER2 Target Fragment (0.06 pmol) | 0.3~3 µL |
| Exnase II | 1 µL |
| 5×CE II buffer | 2 µL |
| Sterilized Deionized Water | 3.6~6.3 µL |
| Total Volume | 10 µL |

Plasmids were extracted using endotoxin-free plasmid extraction reagents, quantified using a spectrophotometer, and sent for sequencing. One day prior to transfection, FreeStyle^{™} 293-F cells were harvested by centrifugation and adjusted to a cell density of 1×10⁶ cells/mL, with the transfection ratio set as cell suspension to medium = 20:1; the concentration of polyethylenimine lineae (PEI) was 2 µL /mL, and the concentration of plasmid was 1 µg/mL; specifically, 40 µg of plasmid was dissolved in 2 mL of Opti-MEM medium and mixed gently, followed by the addition of 80 µL of PEI transfection reagent. The mixture was vortexed for 10 s to ensure thorough mixing and then allowed to stand at room temperature for 10-15 min. The 2 mL mixed solution was added to 40 mL of well-conditioned FreeStyle^{™} 293-F cells and mixed gently, followed by shaker flask culture at 37 °C, 120 rpm and 5% CO₂ for 3-4 days. The supernatant was collected for SDS-PAGE analysis. Furthermore, Protein A affinity purification was performed on a protein purification system, and the sample was collected by elution with 0.1 M citric acid-sodium citrate buffer.

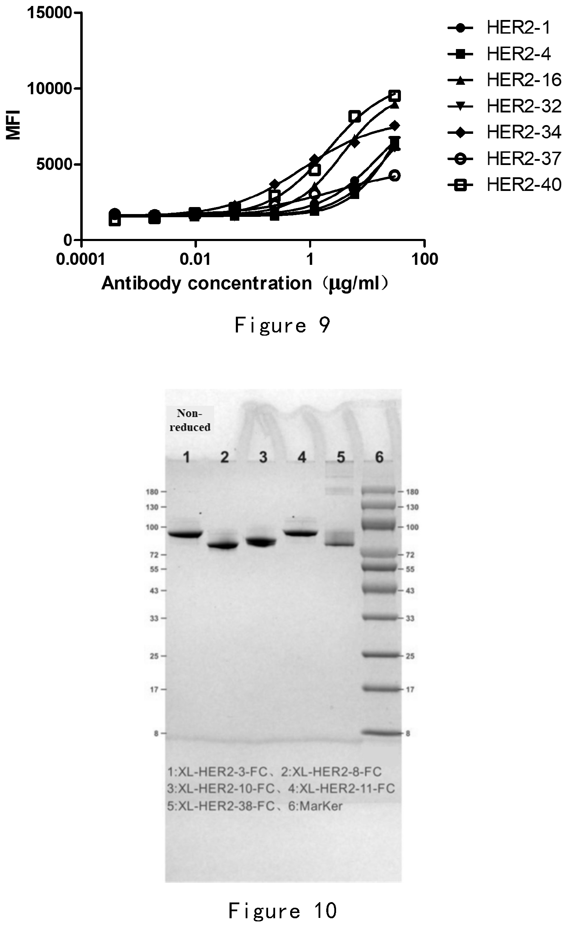

As shown in Figure 10, the results indicated that the molecular weight of the humanized single-domain antibody Fc fusion protein revealed by protein electrophoresis was consistent with the expected value.

### 2.7 Preparation of Trastuzumab

The light chain and heavy chain genes of Trastuzumab were synthesized by gene synthesis:
Trastuzumab light chain: 
DIQMTQSPSSLSASVGDRVTITCRASQDVNTAVAWYQQKPGKAPKLLIY
SASFLYSGVPSRFSGSRSGTDFTLTISSLQPEDFATYYCQQHYTTPPTFGQGTK
VEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTK
SFNRGEC (SEQ ID NO: 23)
Trastuzumab heavy chain:

EVQLVESGGGLVQPGGSLRLSCAASGFNIKDTYIHWVRQAPGKGLEWV
ARIYPTNGYTRYADSVKGRFTISADTSKNTAYLQMNSLRAEDTAVYYCSRW
GGDGFYAMDYWGQGTLVTVSSASTKGPSVFPLAPSSKSTSGGTAALGCLVK
DYFPEPVTVSWNSGALTSGVHTFPAVLQSSGLYSLSSVVTVPSSSLGTQTYIC
NVNHKPSNTKVDKKVEPKSCDKTHTCPPCPAPELLGGPSVFLFPPKPKDTLMI
SRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSR
EEMTKNQVSLTCLVKGFYPSDIAVEWESNGQPENNYKTTPPVLDSDGSFFLY
SKLTVDKSRWQQGNVFSCSVMHEALHNHYTQKSLSLSPGK (SEQ ID NO: 24)

The genes were subcloned into the eukaryotic expression vector pcDNA3.1. The well-conditioned FreeStyle^{™} 293-F cells were co-transfected with the light chain and heavy chain, followed by shaker flask culture at 37 °C, 120 rpm and 5% CO₂ for 3.5 days. The supernatant was harvested and purified using a Protein A column, with elution performed using 0.1 M citric acid-sodium citrate buffer. Buffer exchange and concentration were carried out using ultrafiltration concentrator tubes, and the protein purity was determined by SDS-PAGE.

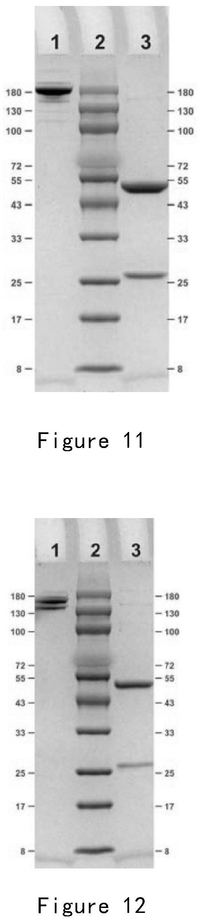

As shown in Figure 11, the Trastuzumab protein identification results demonstrated that the molecular weight of the protein was consistent with the expected value and the purity was high.

Furthermore, the protein concentration was quantified using a spectrophotometer, with a concentration of 1 mg/mL.

### 2.8 Preparation of Pertuzumab

The light chain and heavy chain genes of Pertuzumab were synthesized by gene synthesis:
Pertuzumab light chain: 
DIQMTQSPSSLSASVGDRVTITCKASQDVSIGVAWYQQKPGKAPKLLIY
SASYRYTGVPSRFSGSGSGTDFTLTISSLQPEDFATYYCQQYYIYPYTFGQGT
KVEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPVT
KSFNRGEC (SEQ ID NO: 25)
Pertuzumab heavy chain:

EVQLVESGGGLVQPGGSLRLSCAASGFTFTDYTMDWVRQAPGKGLEW
VADVNPNSGGSIYNQRFKGRFTLSVDRSKNTLYLQMNSLRAEDTAVYYCAR
NLGPSFYFDYWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICN
VNHKPSNTKVDKKVEPKSCDKTHTCPPCPAPELLGGPSVFLFPPKPKDTLMIS
RTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRE

EMTKNQVSLTCLVKGFYPSDIAVEWESNGQPENNYKTTPPVLDSDGSFFLYS
KLTVDKSRWQQGNVFSCSVMHEALHNHYTQKSLSLSPGK (SEQ ID NO: 26)

The genes were subcloned into the eukaryotic expression vector pcDNA3.1. The well-conditioned FreeStyle^{™} 293-F cells were co-transfected with the light chain and heavy chain, followed by shaker flask culture at 37 °C, 120 rpm and 5% CO₂ for 3.5 days. The supernatant was harvested and purified using a Protein A column, with elution performed using 0.1 M citric acid-sodium citrate buffer. Buffer exchange and concentration were carried out using ultrafiltration concentrator tubes, and the protein purity was determined by SDS-PAGE.

As shown in Figure 12, the Pertuzumab protein identification results demonstrated that the molecular weight of the protein was consistent with the expected value and the purity was high.

Furthermore, the protein concentration was quantified using a spectrophotometer, with a concentration of 1 mg/mL.

### 2.9 Detection of the Binding of Candidate Anti-HER2-Fc Single-Domain Antibody Fusion Proteins to Human HER2 Protein

ELISA plates were coated with human HER2-His protein at a concentration of 200 ng per well and incubated overnight at 4 °C. Subsequently, the single-domain antibody fusion proteins obtained in Example 2.6 (with Trastuzumab as the positive control) were subjected to 12 serial 3-fold dilutions starting from 20 µg/mL. The plates were blocked at 37 °C for 1 h, and then the blocked full antibodies were added at 100 µL per well, followed by static incubation at 37 °C for 1 h. After washing, HRP-conjugated anti-human IgG tag antibody was added at 100 µL per well and incubated statically at 37 °C for 45 min. Following another round of washing, TMB substrate chromogenic solution was added at 100 µL per well and incubated statically at room temperature for 10 min. The reaction was terminated by adding 100 µL of 1 M H₂SO₄ per well. The absorbance was measured at a wavelength of 450 nm using a microplate reader. Data processing and parameter fitting were performed using GraphPad Prism Software 5.0 to calculate the EC₅₀ values.

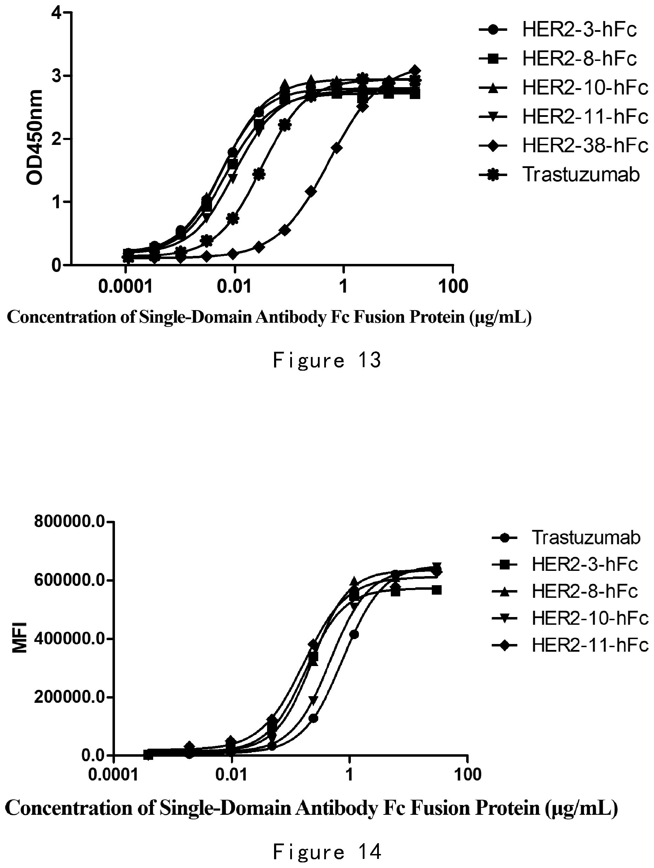

The binding activities of the 5 purified single-domain antibody Fc fusion proteins to HER2 protein were analyzed by ELISA. As shown in Figure 13, the results demonstrated that HER2-3-hFc, HER2-8-hFc, HER2-10-hFc and HER2-11-hFc exhibited superior binding activity compared with the control antibody Trastuzumab, with typical dose-dependent characteristics.

The EC₅₀ values are shown in Table 11 below:

**Table 11**

| Name | HER2-3-hFc | HER2-8-hFc | HER2-10-hFc | HER2-11-hFc | HER2-38-hFc | Trastuzumab |
|---|---|---|---|---|---|---|
| EC₅₀ | 0.005864 | 0.006985 | 0.006238 | 0.009997 | 0.5382 | 0.03054 |

### 2.10 Detection of Binding Activity Between Anti-HER2 Single-Domain

### Antibody Fc Fusion Proteins and SKBR3 Cells by FACS Method

SKBR3 cells were cultured to the logarithmic growth phase, resuspended in FACS buffer, and adjusted to a cell density of 3×10⁶ cells/mL, with 100 µL of the cell suspension added to each well. Subsequently, the single-domain antibody Fc fusion proteins obtained in Example 2.6 (with Trastuzumab as the positive control) were subjected to serial 5-fold dilutions starting from an initial concentration of 30 µg/mL using FACS buffer. The cells were resuspended with 100 µL of the diluted primary antibody per well and incubated at 4 °C for 60 min. After washing three times with PBS, the cells were resuspended with the secondary antibody, anti-Human IgG, Fc Fragment (Alexa Fluor 488-conjugated AffiniPure) and incubated at 4 °C for 60 min. The cells were washed three times with PBS again, then resuspended with 100 µL of PBS per well. The mean fluorescence intensity (MFI) was detected using a Beckman CytoFLEX flow cytometer. Data processing and parameter fitting were performed using GraphPad Prism Software 5.0 to calculate the EC₅₀ values.

As shown in Figure 14, the results demonstrated that at the cellular level, HER2-3-hFc, HER2-8-hFc, HER2-10-hFc and HER2-11-hFc exhibited superior binding activity compared with Trastuzumab.

The EC₅₀ values are shown in Table 12 below:

**Table 12**

| Name | Trastuzumab | HER2-3-hFc | HER2-8-hFc | HER2-10-hFc | HER2-11-hFc |
|---|---|---|---|---|---|
| EC₅₀ | 0.7676 | 0.1788 | 0.2314 | 0.4685 | 0.1649 |

### 2.11 Analysis of Binding Specificity of Anti-HER2 Single-Domain Antibody Fc Fusion Proteins to HER2 Protein

Different proteins were coated at 200 ng per well, including the antigens HER2, PDL1, BCMA, CD38, TSLP, IL-18, IL-15, IL-23, HAS, BSA, TNF-α and IL-4, all of which were stored in the inventors' laboratory. The plates were incubated overnight at 4 °C. Then, the antibodies HER2-3-Fc, HER2-8-Fc, HER2-10-Fc and HER2-11-Fc were added at a dosage of 0.2 µg per well for replicate well detection, followed by static incubation at 37 °C for 1 h. After washing, HRP-conjugated anti-IgG tag antibody was added to the ELISA plates at 100 µL per well and incubated statically at 37 °C for 45 min. Following another round of washing, TMB substrate chromogenic solution was added, and the color reaction was terminated with 1 M H₂SO₄. The absorbance was measured at a wavelength of 450 nm using a microplate reader. Data processing was performed using GraphPad Prism Software 5.0.

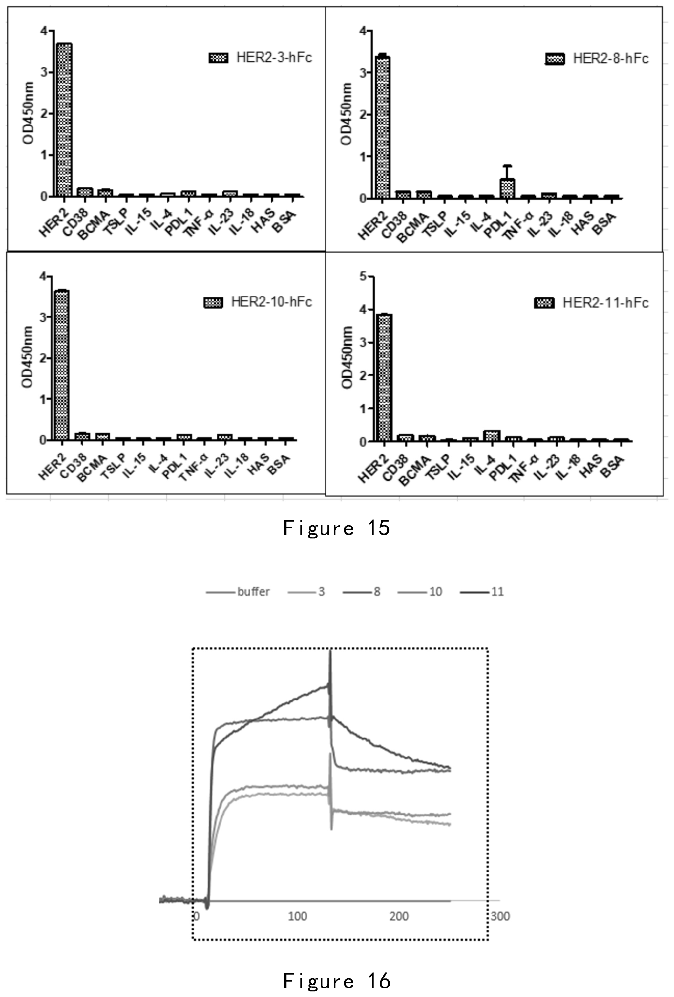

As shown in the specificity assay results in Figure 15, HER2-3-Fc, HER2-8-Fc, HER2-10-Fc and HER2-11-Fc bound to human HER2 protein with high specificity and affinity, and no cross-reactivity with other tested proteins was observed.

### 2.12 Epitope Analysis of Anti-HER2 Single-Domain Antibodies, Trastuzumab and Pertuzumab

The capture antibody was immobilized onto the surface of CM5 chips using the Human Antibody Capture Kit, and the kinetic parameters of antibody-antigen-antibody interactions were determined via the multi-cycle kinetics approach. Purified HER2-His and Trastuzumab/Pertuzumab were separately diluted to 1 µg/mL with HBS-EP buffer. Trastuzumab or Pertuzumab was captured and immobilized onto the chip surface at 25 °C with a flow rate of 5 µL/min for 1 min, with the target capture response value reaching approximately 400 RU. HER2-His antigen was allowed to bind for 100 s. The single-domain antibodies HER2-3, HER2-8, HER2-10 and HER2-11 were diluted to 1.5 µg/mL with HBS-EP buffer and used as the mobile phase. The assay conditions were set as follows: temperature at 25 °C, flow rate at 30 µL/min, association time of 120 s, and dissociation time of 120 s. The regeneration conditions were 10 mM glycine-hydrochloric acid buffer (pH 2.5) at a flow rate of 30 µL/min for 90 s. Data analysis was subsequently performed.

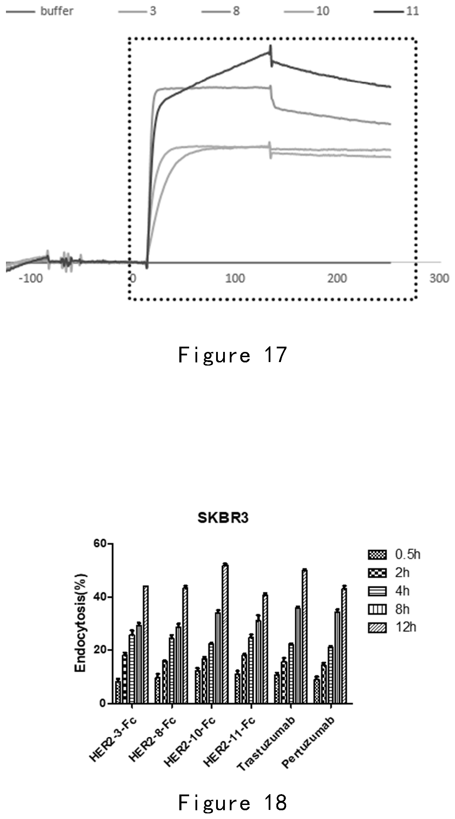

As shown in the epitope validation assay results in Figures 16 and 17, the epitopes recognized by the single-domain antibodies HER2-3-Fc, HER2-8-Fc, HER2-10-Fc and HER2-11-Fc were distinct from those recognized by Trastuzumab and Pertuzumab.

### 2.13 Endocytosis Assay

For the endocytosis assay, SKBR3 cells/NCI-N87 cells were seeded at 2×10⁵ cells per well (the cell density was adjusted to 4×10⁶ cells/mL and diluted with medium, with 100 µL added per well). Then, 50 µL of the antibody solution was added to each well. The antibodies HER2-3, HER2-8, HER2-10, HER2-11, Trastuzumab and Pertuzumab were separately added at a concentration of 20 µg/mL (50 µL per well, equivalent to 1 µg per well). The cells were incubated on ice at 4 °C for 30 min, followed by incubation at 37 °C for 0 h, 30 min, 1 h, 2 h, 4 h, 10 h and 24 h. After incubation, the cells were centrifuged at 1500 rpm for 5 min, washed three times with PBS, and resuspended in 200 µL of PBS per well. The anti-Fc-FITC secondary antibody, diluted 1:200 with FACS buffer containing 2% serum, was added at 100 µL per well, and the cells were incubated at 4 °C for 1 h. The cells were centrifuged again at 1500 rpm for 5 min, washed three times with PBS, resuspended in 200 µL of PBS per well, and finally resuspended in 100 µL of FACS buffer. The endocytosis efficiency at different time points was detected by flow cytometry.

**Table 13**

| Mean Endocytosis Rate Data of SKBR3 Cells | | | | | |
|---|---|---|---|---|---|
| | 0.5h | 2h | 4h | 8h | 12h |
| HER2-3-Fc | 8% | 18% | 26% | 29% | 44% |
| HER2-8-Fc | 10% | 16% | 24% | 29% | 43% |
| HER2-10-Fc | 12% | 17% | 23% | 34% | 52% |
| HER2-11-Fc | 11% | 18% | 25% | 31% | 41% |
| Trastuzumab | 11% | 16% | 22% | 36% | 50% |
| Pertuzumab | 9% | 14% | 21% | 35% | 43% |

As shown in Figure 18 and Table 13, HER2-10-Fc exhibited a higher endocytosis rate, making it a preferred sequence for antibody-drug conjugate (ADC) development. HER2-3-Fc, HER2-8-Fc and HER2-11-Fc showed relatively lower endocytosis efficiency, and thus can serve as targeting proteins for tumor therapeutic applications.

All literatures mentioned in the present invention are incorporated herein by reference, as though each one is individually incorporated by reference. In addition, it should be understood that, after reading the above teachings of the present invention, those skilled in the art can make various changes or modifications to the present invention, these equivalents also fall within the scope as defined in the appended claims of the present application.

## Claims

1. An anti-HER2 single-domain antibody, wherein the anti-HER2 single-domain antibody has one or more complementarity-determining regions (CDRs) selected from the group consisting of:
(1) CDR1 as set forth in SEQ ID NO: 27, CDR2 as set forth in SEQ ID NO: 28, and CDR3 as set forth in SEQ ID NO: 29;
(2) CDR1 as set forth in SEQ ID NO: 34, CDR2 as set forth in SEQ ID NO: 35, and CDR3 as set forth in SEQ ID NO: 36;
(3) CDR1 as set forth in SEQ ID NO: 40, CDR2 as set forth in SEQ ID NO: 41, and CDR3 as set forth in SEQ ID NO: 42;
(4) CDR1 as set forth in SEQ ID NO: 46, CDR2 as set forth in SEQ ID NO: 47, and CDR3 as set forth in SEQ ID NO: 48;
(5) CDR1 as set forth in SEQ ID NO: 52, CDR2 as set forth in SEQ ID NO: 53, and CDR3 as set forth in SEQ ID NO: 54;
(6) CDR1 as set forth in SEQ ID NO: 57, CDR2 as set forth in SEQ ID NO: 58, and CDR3 as set forth in SEQ ID NO: 59;
(7) CDR1 as set forth in SEQ ID NO: 63, CDR2 as set forth in SEQ ID NO: 64, and CDR3 as set forth in SEQ ID NO: 65;
(8) CDR1 as set forth in SEQ ID NO: 69, CDR2 as set forth in SEQ ID NO: 70, and CDR3 as set forth in SEQ ID NO: 71;
(9) CDR1 as set forth in SEQ ID NO: 74, CDR2 as set forth in SEQ ID NO: 75, and CDR3 as set forth in SEQ ID NO: 76;
(10) CDR1 as set forth in SEQ ID NO: 27, CDR2 as set forth in SEQ ID NO: 28, and CDR3 as set forth in SEQ ID NO: 80;
(11) CDR1 as set forth in SEQ ID NO: 83, CDR2 as set forth in SEQ ID NO: 84, and CDR3 as set forth in SEQ ID NO: 85;
(12) CDR1 as set forth in SEQ ID NO: 57, CDR2 as set forth in SEQ ID NO: 58, and CDR3 as set forth in SEQ ID NO: 89;
(13) CDR1 as set forth in SEQ ID NO: 91, CDR2 as set forth in SEQ ID NO: 92, and CDR3 as set forth in SEQ ID NO: 93;
(14) CDR1 as set forth in SEQ ID NO: 97, CDR2 as set forth in SEQ ID NO: 98, and CDR3 as set forth in SEQ ID NO: 99.

2. The anti-HER2 single-domain antibody of claim 1, wherein the anti-HER2 single-domain antibody further comprises framework regions (FRs), and the framework regions (FRs) are one or more FRs selected from the group consisting of:
(1) FR1 as set forth in SEQ ID NO: 30, FR2 as set forth in SEQ ID NO: 31, FR3 as set forth in SEQ ID NO: 32 and FR4 as set forth in SEQ ID NO: 33;
(2) FR1 as set forth in SEQ ID NO: 37, FR2 as set forth in SEQ ID NO: 38, FR3 as set forth in SEQ ID NO: 39 and FR4 as set forth in SEQ ID NO: 33;
(3) FR1 as set forth in SEQ ID NO: 43, FR2 as set forth in SEQ ID NO: 44, FR3 as set forth in SEQ ID NO: 45 and FR4 as set forth in SEQ ID NO: 33;
(4) FR1 as set forth in SEQ ID NO: 37, FR2 as set forth in SEQ ID NO: 49, FR3 as set forth in SEQ ID NO: 50 and FR4 as set forth in SEQ ID NO: 33;
(5) FR1 as set forth in SEQ ID NO: 51, FR2 as set forth in SEQ ID NO: 31, FR3 as set forth in SEQ ID NO: 32 and FR4 as set forth in SEQ ID NO: 33;
(6) FR1 as set forth in SEQ ID NO: 37, FR2 as set forth in SEQ ID NO: 55, FR3 as set forth in SEQ ID NO: 56 and FR4 as set forth in SEQ ID NO: 33;
(7) FR1 as set forth in SEQ ID NO: 60, FR2 as set forth in SEQ ID NO: 61, FR3 as set forth in SEQ ID NO: 62 and FR4 as set forth in SEQ ID NO: 33;
(8) FR1 as set forth in SEQ ID NO: 66, FR2 as set forth in SEQ ID NO: 67, FR3 as set forth in SEQ ID NO: 68 and FR4 as set forth in SEQ ID NO: 33;
(9) FR1 as set forth in SEQ ID NO: 37, FR2 as set forth in SEQ ID NO: 72, FR3 as set forth in SEQ ID NO: 73 and FR4 as set forth in SEQ ID NO: 33;
(10) FR1 as set forth in SEQ ID NO: 77, FR2 as set forth in SEQ ID NO: 78, FR3 as set forth in SEQ ID NO: 79 and FR4 as set forth in SEQ ID NO: 33;
(11) FR1 as set forth in SEQ ID NO: 30, FR2 as set forth in SEQ ID NO: 81, FR3 as set forth in SEQ ID NO: 82 and FR4 as set forth in SEQ ID NO: 33;
(12) FR1 as set forth in SEQ ID NO: 86, FR2 as set forth in SEQ ID NO: 87, FR3 as set forth in SEQ ID NO: 88 and FR4 as set forth in SEQ ID NO: 33;
(13) FR1 as set forth in SEQ ID NO: 30, FR2 as set forth in SEQ ID NO: 61, FR3 as set forth in SEQ ID NO: 90 and FR4 as set forth in SEQ ID NO: 33;
(14) FR1 as set forth in SEQ ID NO: 94, FR2 as set forth in SEQ ID NO: 95, FR3 as set forth in SEQ ID NO: 96 and FR4 as set forth in SEQ ID NO: 33;
(15) FR1 as set forth in SEQ ID NO: 100, FR2 as set forth in SEQ ID NO: 101, FR3 as set forth in SEQ ID NO: 102 and FR4 as set forth in SEQ ID NO: 33.

3. The anti-HER2 single-domain antibody of claim 1, wherein the amino acid sequence of the VHH chain of the anti-HER2 single-domain antibody is selected from one or more of SEQ ID NOs: 6-20.

4. An anti-HER2 antibody comprising one or more VHH chains of the anti-HER2 single-domain antibody of any one of claims 1-3.

5. The anti-HER2 antibody of claim 4, wherein the anti-HER2 antibody comprises a monomer, a bivalent antibody, and/or a multivalent antibody.

6. A chimeric antigen receptor (CAR), wherein the CAR comprises an extracellular domain that comprises the anti-HER2 single-domain antibody of any one of claims 1-3, or the anti-HER2 antibody of any one of claims 4-5.

7. A fusion protein, comprising:
(Z1) a first protein, wherein the first protein comprises: the anti-HER2 single-domain antibody of any one of claims 1-3, the anti-HER2 antibody of any one of claims 4-5, or an active fragment thereof;
(Z2) a second protein, wherein the second protein comprises a cytokine; and
(Z3) an optional linker located between the first protein and the second protein.

8. A recombinant protein, comprising:
(i) the anti-HER2 single-domain antibody of any one of claims 1-3, the anti-HER2 antibody of any one of claims 4-5, the fusion protein of claim 7, or an active fragment thereof; and
(ii) an optional tag sequence that facilitates expression and/or purification.

9. A polynucleotide, wherein the polynucleotide encodes a protein selected from the group consisting of: the anti-HER2 single-domain antibody of any one of claims 1-3, the anti-HER2 antibody of any one of claims 4-5, the chimeric antigen receptor of claim 6, the fusion protein of claim 7, the recombinant protein of claim 8, and combinations thereof.

10. An expression vector comprising the polynucleotide of claim 9.
